(19) Europäisches Patentamt
European Patent Office
Office européen des brevets

(11) **EP 1 232 286 B1**

(12) **EUROPÄISCHE PATENTSCHRIFT**

(45) Veröffentlichungstag und Bekanntmachung des Hinweises auf die Patenterteilung:
**28.05.2008 Patentblatt 2008/22**

(21) Anmeldenummer: **00985048.8**

(22) Anmeldetag: **16.11.2000**

(51) Int Cl.:
***C12Q 1/68*** (2006.01)

(86) Internationale Anmeldenummer:
**PCT/EP2000/011386**

(87) Internationale Veröffentlichungsnummer:
**WO 2001/036673 (25.05.2001 Gazette 2001/21)**

(54) **TEST AUF MIKROORGANISMEN ZUR DIAGNOSE VON SEPSIS**

TEST FOR MICRO-ORGANISMS FOR THE DIAGNOSIS OF SEPSIS

TEST POUR MICRO-ORGANISMES POUR LE DIAGNOSTIC DE SEPSIS

(84) Benannte Vertragsstaaten:
**AT BE CH CY DE DK ES FI FR GB GR IE IT LI LU MC NL PT SE TR**

(30) Priorität: **17.11.1999 DE 19955303**

(43) Veröffentlichungstag der Anmeldung:
**21.08.2002 Patentblatt 2002/34**

(73) Patentinhaber: **Izinta Trading Co. Ltd.**
**1121 Budapest (HU)**

(72) Erfinder:
• **APFEL, Heiko**
**86356 Neusäss (DE)**
• **HEESEMANN, Jürgen**
**80639 München (DE)**
• **TREBESIUS, Karlheinz**
**83093 Bad Endorf (DE)**
• **AUTENRIETH, Ingo**
**72020 Tübingen (DE)**

(74) Vertreter: **Weiss, Wolfgang et al**
**Weickmann & Weickmann**
**Patentanwälte**
**Kopernikusstrasse 9**
**81679 München (DE)**

(56) Entgegenhaltungen:
**WO-A-99/54502**

• **DATABASE EMBL [Online] EBI; 19. Juni 1997 (1997-06-19) EMLER S ET AL.: "Streptococcus parasanguis 16S ribosomal RNA gene" retrieved from HTTP://WWW.EBI.AC.UK Database accession no. AF003933 XP002265998**
• **DATABASE EMBL [Online] EBI; 16. März 1999 (1999-03-16) BARASH SC ET AL.: "Staphylococcus aureus contig SEQ ID #5025" retrieved from HTTP://WWW.EBI.AC.UK Database accession no. AAV79336 XP002265999**
• **DATABASE EMBL [Online] EBI; 6. Oktober 1999 (1999-10-06) WANG C ET AL.: "Sequence 345 from patent US 5861244" retrieved from HTTP://WWW.EBI.AC.UK Database accession no. AR030156 XP002266000**
• **NEEF A ET AL: "Detection of sphingomonads and in situ identification in activated sludge using 16S rRNA-targeted oligonucleotide probes" JOURNAL OF INDUSTRIAL MICROBIOLOGY AND BIOTECHNOLOGY, Bd. 23, Nr. 4-5, Oktober 1999 (1999-10), Seiten 261-267, XP002265997 ISSN: 1367-5435**
• **GORY L ET AL: "IDENTIFICATION OF STAPHYLOCOCCUS CARNOSUS AND STAPHYLOCOCCUS WARNERI ISOLATED FROM MEAT BY FLUORESCENT IN SITU HYBRIDIZATION WITH 16S RRNA-TARGETED OLIGONUCLEOTIDE PROBES" SYSTEMATIC AND APPLIED MICROBIOLOGY, XX, XX, Bd. 22, Nr. 2, Mai 1999 (1999-05), Seiten 225-228, XP001027219 ISSN: 0723-2020**

**Beschreibung**

Die vorliegende Erfindung betrifft einen neuheitlichen Ansatz zur direkten Identifizierung von Keimen in Probenmaterial. Im erfindungsgemäßen Verfahren wird entsprechend der Fragestellung eine ausgewählte Sondengruppe eingesetzt, die mit hoher Wahrscheinlichkeit alle relevanten Keime im Probenmaterial erfasst. Die ausgewählte Sondengruppe identifiziert mit hoher Spezifität die jeweiligen Keime im Probenmaterial, wobei die einzelnen Sonden vorzugsweise gegen bestimmte Bereiche innerhalb intakter Ribosomen gerichtet sind.

Die schnelle und sichere Identifizierung von Mikroorganismen in biologischen Proben spielt eine essentielle Rolle in der medizinischen und industriellen Mikrobiologie, z.B. in der Lebensmittel- und Pharmaindustrie. In beiden Sparten kann eine schnelle und sichere Identifizierung schädlicher Mikroorganismen zu enormen Kosteneinsparungen im Gesundheitssystem bzw. bei industriellen Produktionsprozessen führen. Gleiches gilt für die Identifizierung nützlicher Mikroorgansimen im Sinne einer Qualitätskontrolle, z.B. bei Produktionsprozessen. In der medizinischen Mikrobiologie spielen schnelle Verfahren zu Identifizierung mikrobieller Krankheitserreger, insbesondere bei fulminanten und nosokomialen Infektionen eine wichtige Rolle. In diesen besonderen Fällen muß die Identifzierung des Krankheitserregers innerhalb eines Tages abgeschlossen sein, damit das Untersuchungsergebnis auf die Therapie einen Einfluß hat. Dies ist in der gängigen Praxis zur Zeit nur sehr eingeschränkt möglich. Als direkte Folge werden die betroffenen Patienten gemäß ärztlicher Einschätzung mit Breitband-Antibiotika therapiert. Diese Therapie ist für den Patienten mit starken Nebenwirkungen verbunden. Insbesondere führt die massive Anwendung dieser Therapie zu einer kontinuierlichen Zunahme an Keimen, die gegen das Mittel eine Resistenz entwickeln (s. dazu, Bericht an amerikanischen Kongress).

Die bislang in der medizinischen Routine angewandten Verfahren zur Identifizierung von Mikroorganismen in klinischen Proben umfassen die klassische Kultur sowie serologische Verfahren. Verfahren, die auf Nukleinsäuren abzielen, wie Amplifikations- (PCR) und Hybridisierungsmethoden werden bislang nur für Spezialuntersuchungen eingesetzt.

Die traditionellen Nachweismethoden umfassen die Kultur der Mikroorganismen, mikroskopische Methoden und serologische Antigen-Antikörper-Verfahren (s. dazu DGHM, Verfahrensrichtlinien für die mikrobiologische Diagnostik, Gustav Fischer Verlag). Zur Kultivierung gibt es eine große Anzahl verschiedener Wachstumstests, die auf das Wachstum eines bestimmten Organismus oder einer Organismen-Gruppe abgestimmt sind. Diese Methode ist sehr sensitiv, da theoretisch ein einziger pathogener Organismus, der sich in einer biologischen Probe befindet, nachgewiesen werden kann. Sie ist jedoch sehr zeitaufwendig und daher für die oben genannten Anwendungsbereiche, in denen eine schnelle Identifizierung der Mikroorganismen notwendig ist, nur eingeschränkt geeignet. Die Standardkultur z.B. von schnellwachsenden Bakterien dauert zwischen 18 bis 24 Stunden, die von Pilzen schon mindestens 48 Stunden und die von speziellen Mikroorganismen bis zu mehreren Tagen bzw. Wochen. Nach erfolgreicher Kultivierung schließt sich in der Regel noch eine mindestens 24-stündige Differenzierungsphase (z.B. "bunte Reihe") an. Außerdem können manche Organismen gar nicht angezüchtet werden, da ihre spezifischen Kulturbedingungen nicht bekannt sind. Weiterhin wachsen Bakterien, die in der Anfangsphase einer Antibiotikatherapie isoliert werden, oftmals nicht an. Auch Mikroorganismen, die sich in die sogenannte "viable but not culturable" (VNC)-Form, eine Überdauerungsform, die sich später *in vivo* wieder redifferenziert, umgewandelt haben, können nicht mehr kultiviert werden.

Mikroskopische Nachweisverfahren sind in der Regel mit Färbeverfahren gekoppelt, z.B. der Gram-Färbung. Diese Verfahren liefern relativ rasch Ergebnisse, sofern der Mikroorganismus in der klinischen Probe in größerer Anzahl vorliegt. Allerdings besitzen diese Verfahren keine hohe Spezifität. Ein spezifischer Nachweis des relevanten Krankheitserregers in Anwesenheit vieler anderer Mikrooganismen ist kaum möglich, z.B. die Identifizierung eines bestimmten Typs Gram-negativer Stäbchen in Anwesenheit anderer Gram-negativer Stäbchen.

Serologische Nachweisverfahren für Mikroorganismen werden in der Routine nur in Einzelfällen eingesetzt. Die Herstellung von Antiseren, die Mikroorganismen mit hoher Spezifität identifizieren, ist sehr teuer. Darüber hinaus ist die Durchführung des Nachweisverfahrens sehr zeitaufwendig, da in der Regel eine Kultivierung der Probe zur Anreicherung des Mikroorganismus erforderlich ist, um die relativ hohe Empfindlichkeitsgrenze des Verfahrens zu überschreiten.

Neuere Verfahren zur Identifizierung von Mikroorganismen zielen auf die Nukleinsäuren ab, wie DNA und RNA. Hierzu zählen Hybridisierungsmethoden sowie enzymatische Amplifikationsmethoden, wie z.B. die Polymerase Ketten Reaktion, kurz PCR genannt.

Unter Nukleinsäurehybridisierung versteht man insbesondere die Bildung einer Doppelhelix aus zwei einzelsträngigen, komplementären Poly- oder Oligonukleotiden. Diese Poly- oder Oligonukleotide können z.B. zwei einzelsträngige DNA-Moleküle, ein einzelsträngiges DNA- und ein einzelsträngiges RNA-Molekül oderzwei einzelsträngige RNA-Moleküle sein. Auch Nukleotidanaloga wie peptidische Nukleinsäuren (PNA) sind in der Lage, miteinander oder mit Nukleinsäuren Hybride zu bilden. Die Nukleinsäurehybridisierung wird dazu verwendet, eine sehr kleine oder aber auch eine sehr große Anzahl an Basen einer bestimmten Sequenzfolge innerhalb einer Nukleinsäure, der Ziel-Sequenz in Anwesenheit einer Vielzahl andersartiger Sequenzfolgen (Nicht-Zielsequenzen) nachzuweisen. Die Hybridisierungsreaktion kann entweder in Lösung oder mit Nukleinsäuren, die vorher immobilisiert werden, erfolgen. Die Kinetik einer Hybridisierung wird direkt von der Konzentration der nachzuweisenden Nukleinsäure, dem Target und der verwendeten Sonde beeinflußt, die zu einer bestimmten Sequenzfolge innerhalb der Nukleinsäure komplementär ist. Auch die Temperatur und die molekulare Zusammensetzung der Lösung, in der die Hybridisierungsreaktion abläuft, haben einen direkten Einfluß auf die Kinetik einer Hybridisierung (49).

Die ribosomale RNA ist ein wesentlicher Bestandteil des Syntheseapparats einer Zelle. Diese Nukleinsäure liegt bei aktiven Zellen in sehr hoher Kopienzahl vor und stellt somit ein ideales Zielmolekül für Hybridisierungsverfahren dar. Bereits 1971 konnten Pace und Campbell zeigen, daß die rRNA verschiedener bakterieller Spezies homolog ist und die Unterschiede zwischen den verschiedenen Spezies mit Hilfe von Hybridisierungstechniken quantifizierbar sind (27). Sogin und Woese diskutieren 1972 die theroretischen und praktischen Aspekte der Verwendung von rRNA-Primärsequenzen zur Ableitung phylogenetischer Verwandtschaftsbeziehungen (41). Fox, Pechman und Woese erstellten 1977 durch vergleichende Analyse von 16S rRNA Oligonukleotidsequenzen ein natürliches System für Prokaryonten (12).

Die Empfindlichkeit der Hybridisierungstechniken, z.B. zur Identifizierung von Mikroorganismen, hängt u.a. vom Verhältnis Ziel-Sequenz zu Nicht-Ziel-Sequenz ab. Handelt es sich bei der Ziel-Nukleinsäure um chromosomale DNA, so ist dieses Verhältnis sehr gering. Eine Verbesserung des Verhältnisses ist bei RNA, insbesondere jedoch bei ribosomaler RNA, auch als rRNA bezeichnet, zu erwarten. Allerdings liegt die rRNA innerhalb eines Proteinkomplexes vor, dem Ribosom, der die Zugänglichkeit dieser Ziel-Nukleinsäure stark einschränkt. Diese natürliche Einschränkung kann durch die Extraktion der rRNA teilweise aufgehoben werden. Hierzu muß die Ziel-Zelle lysiert und die Ziel-Nukleinsäure von gebundenen Komponenten, insbesondere Proteinen, befreit werden, so daß sie für die Hybridisierung frei verfügbar ist. Die von Proteinen befreite Ziel-Nukleinsäure wird in einem zweiten Schritt auf einen Träger immobilisiert und steht somit den verschiedenen Schritten einer Hybridisierungsreaktion zur Verfügung. Wenngleich dieses allgemein angewandte Verfahren vom theoretischen Ablauf eine Routinefähigkeit besitzt, zeichnen sich in der Praxis weitere Einschränkungen ab, die sich auf die Empfindlichkeit des Nachweistests auswirken. Wird dieses Verfahren z.B. an klinischen Proben, wie Gewebematerial, durchgeführt, so wird bei der Lyse nicht nur die Ziel-Nukleinsäure der Zielzelle freigesetzt, sondern auch die (Nicht-)Ziel-Nukleinsäure der Gewebezellen, d.h. das Verhältnis von Ziel-Sequenz zu Nicht-Ziel-Sequenz wird massiv zu geringeren Werten verschoben. Liegen im untersuchten Gewebematerial nur sehr wenige Ziel-Zellen vor, sind die Ziel-Zellen mit dem Verfahren nicht mehr nachzuweisen. Folgende Lösungswege wurden eingeschlagen.

Der einfachste Lösungsweg ist die gezielte Anzüchtung der Ziel-Zellen vor dem Hybridisierungsverfahren. Oftmals werden in der Praxis einzelne Zellkolonien dem Nachweisverfahren zugeführt. Sicherlich kann auf diesem Weg eine hohe Empfindlichkeit erzielt werden, allerdings ist diese Vorgehensweise sehr zeitaufwendig, da ein bis zwei Kultivierungsschritte benötigt werden.

Ein weiterer Lösungsweg beinhaltet eine Vervielfältigung, Amplifikation, der entsprechenden Ziel-Nukleinsäure bzw. eines entsprechenden Teils davon. In diesem Fall ist ebenfalls eine Lyse der Ziel-Zelle erforderlich. D.h. die oben dargestellte Problematik kommt zum Tragen. Die Amplifikation der Ziel-Nukleinsäure bzw. eines Teils davon wird mittels PCR durchgeführt und erfordert den Einsatz von Primern, die spezifisch an zwei Ziel-Sequenzen innerhalb der Ziel-Nukleinsäure binden müssen. Handelt es sich bei der Ziel-Nukleinsäure um eine RNA bzw. rRNA, wird zunächst mit Hilfe einer Reversen Transkriptase-Reaktion eine komplementäre DNA hergestellt, die cDNA. Diese cDNA kann dann wiederum als Matrize für die PCR dienen (27). Die Amplifikation von Nukleinsäure-Abschnitten ist auf jeden Fall durch den hohen Wirkungsgrad des Enzyms, der Polymerase, sichergestellt. Das Problem liegt insbesondere darin, die relevanten Abschnitte vervielfältigt zu bekommen. Dies muß durch die spezifische Bindung der Primer an den entsprechenden Teil der Ziel-Nukleinsäure gewährleistet sein. Eine Diskriminierung entsprechender Sequenzen durch das Enzym ist ausgeschlossen. Im Gegenteil, es werden alle Bereiche amplifiziert, wo die Primer binden, auch wenn unvollständige intermediäre Bindungen vorliegen. Naturgemäß kommt es hierbei zu falsch positiven Ergenissen, wenn der Anteil von Nicht-Ziel-Sequenzen hoch ist. Insbesondere jedoch, wenn die Nicht-Ziel-Sequenzen z.B. verwandten Nicht-Ziel-Mikroorganismen stammen. Einen Ausweg stellt die Sandwich-Hybridisierungstechnik dar. Hierbei werden die spezifischen Amplifikationsprodukte mit sogenannten Capture-Molekülen nachgewiesen, die auf einer Trägermatrix immobilisiert sind. Die Verwendung von Capture-Sonden wurde z.B. von Ranki et al. (US 4,486,539), Cape et al. (EP 0 370 694) und Gen-Probe (WO 98/50583) beschrieben. Insgesamt nimmt jedoch die Komplexität des Nachweisverfahrens zu, wodurch sich die Störanfälligkeit und Fehlerrate erhöht und somit die Anwendung des Verfahrens im Routinebetrieb in Frage gestellt werden kann.

Ein Anliegen der vorliegenden Erfindung war es, ein Nachweisverfahren zu entwickeln, das aus einfachen Elementen besteht und innerhalb kurzer Zeit ein sicheres Resultat liefert. Die Freisetzung der Ziel-Nukleinsäure wird als ein wesentlicher Nachteil der beschriebenen Verfahren betrachtet. Einen Ausweg bietet der Einsatz nahezu intakter Zellen, d.h. die Ziel-Zelle wird vor der Hybridisierungsreaktion lediglich permeabilisiert und zwar so, daß die entsprechende Sonde in die Zelle eindringen kann und ihre Ziel-Sequenz innerhalb der Zelle, z.B. dem Mikroorganismus erreicht. Da die im Ansatz befindlichen Ziel-Zellen nicht lysiert werden, ergibt sich eine Anreicherung der Ziel-Sequenz im Inneren der Zelle, wobei Nicht-Ziel-Zellen ausgeklammert bleiben. Dieser positive Effekt wird in der in situ Hybridisierung ausgenutzt, wozu eine mikroskopische Auswertung erforderlich wird. In diesem Fall werden einerseits molekulare und andererseits morphologische Informationen über intakte Zellen geliefert und zwar sowohl über die Ziel-Zellen als auch über die Nicht-Ziel-Zellen. Wird der Permeabilisierungsschritt spezifisch auf die Ziel-Zelle, z.B. den Mikroorganismus, ausgerichtet, so daß Nicht-Ziel-Zellen, z.B. Gewebematerial, weniger häufig für die Sonden zugänglich sind, kann die Anwendung des Verfahrens auch in Reaktionsgefäßen, d.h. ohne mikroskopische Auswertung, erfolgen.

Die erste Ganzzellhybridisierung zur Identifizierung einzelner mikrobieller Zellen wurde 1988 von Giovannoni (16) beschrieben. Dieser verwendete rRNA gerichtete radioaktiv markierte Sonden. Nach der Hybridisierung der ganzen fixierten Zellen, wurde die $^{35}$S-markierte Sonde mittels Autoradiographie detektiert. Bereits ein Jahr später wurden an Stelle von radioaktiv markierten Oligonukleotiden fluoreszenzmarkierte rRNAgerichtete Sonden verwendet, die den Nachweis einzelner bakterieller Zellen ermöglichten (8). Insgesamt ist die Gesamtzellhybridisierung eine schnelle und kostengünstige Methode zum routinemäßigen Nachweis einzelner Zellen in gemischten Populationen (2). Aufgrund der Tatsache, daß die Gesamtzellhybridisierung bisher nur auf Objektträgern durchgeführt wird, kann nur eine begrenzte Anzahl von Probenmaterialien untersucht werden, da die mikroskopische Auswertung, v. a. von Proben, die wenige Zielorganismen und viel Matrix und Kommensalflora enthalten, langwierig und anstrengend ist und oft nur von speziell geschultem Personal durchgeführt werden kann. Allerdings kann die Empfindlichkeit des Verfahrens gesteigert werden. Untersucht man beispielsweise eine Bakteriensuspension mit einer Konzentration von $10^5$ Bakterien/ml und bringt man mit 10 μl dieser Suspension auf einer kreisrunden Fläche mit einem Durchmesser von 1 cm auf, so verteilen sich bei einer Vergrößerung von 1000x (ein Gesichtsfeld entspricht 200 qm$^2$) $10^3$ Bakterien auf 2500 Gesichtsfelder. Man muß also durchschnittlich drei Gesichtsfelder untersuchen um ein Bakterium zu finden. Dieses Rechenbeispiel zeigt, daß das Verfahren seine Grenzen hat und personalintensiv ist; z.B. ist in Blutproben die Konzentration an Krankheitserregern viel niedriger, so daß mehrere Objektträger untersucht werden müßten, um den entsprechenden Ziel-Organismus zu finden.

Eine Verbesserung des Verfahrens im Hinblick seiner Routinefähigkeit kann dadurch erzielt werden, daß die Untersuchung der Proben in Reaktionsgefäßen durchgeführt wird, wobei die Erkennung von positiven Proben, z.B. mit einem Krankheitserreger, automatisch erfolgt und zwar durch apparative Detektionssysteme. Von Cruickshank et al. und Wallner et al. wurden Ganzzellhybridisierungsverfahren vorgestellt, die auf der in Situ-Hybridisierung ganzer bakterieller Zellen in Lösung beruhen. Die Auswertung der sondenvermittelten Fluoreszenz kann dabei entweder mikroskopisch oder mittels Durchflußzytometrie erfolgen (EP0497464). Nachteilig an diesen Verfahren ist allerdings, daß die Zellen nach jedem Verfahrensschritt abzentrifugiert werden müssen, um in einer neuen Lösung aufgenommen werden zu können. Die Kombination aus der sensitiven, spezifischen und schnellen Ganzzellhybridisierung mit einem routinemäßig leicht zu handhabenden Nachweis in Reaktionsgefäßen wie z. B. in Mikrotiterplatten (MTP) bzw. anderen Plastik- oder Glasträgern, wäre demnach das ideale mikrobiologische Diagnostikverfahren. Mikrotiterplatten-basierende Nachweisverfahren sind bislang jedoch ausschließlich bei den dargestellten Sandwich-Hybridisierungsverfahren, ELISAs oder PCR-Amplifikationen bekannt. Eine Gesamtzellhybridisierung, in der das zu untersuchende Probenmaterial in Reaktionsgefäßen wie z. B. Mikrotiterplatten immobilisiert ist, wurde bislang noch nicht beschrieben.

WO 99/54502 beschreibt ein Verfahren zur Detektion oder identifizierung eines Bakteriums in einer Probe umfassend einen Test durch Gramfärbung und danach einen Test mit einer Nukleinsäuresonde durch in-situ-Hybridisierung, wobei die Sonde abhängig vom Ergebnis der Gramfärbung ausgewählt wird.

Shimada et al. (J. Infect. Chemother, 1999, 5:21-31) beschreiben ein Verfahren zur Diagnose von Sepsis, welches eine in-situ-Hybridisierung von einem Neutrophilenisolat mit Sonden einer Länge von etwa 2 bis etwa 7 kb einschließt, um Bakterien zu detektieren. Die Hybridisierung wird nach einem bakteriolytischen Schritt durchgeführt.

Das Anliegen der vorliegenden Erfindung war es deshalb ein kulturunabhängiges Verfahren auf der Basis der Gesamtzellhybridisierung zu entwickeln, das schnell, spezifisch, einfach und billig ist. Es enthält keinen lytischen Schritt, ist sensitiv und einfach auszuwerten. Ein weiterer, wesentlicher Anspruch ist, daß das Verfahren z.B. die Untersuchung eines definierten Krankheitsbildes ermöglicht, d.h. nicht nur auf einen einzelnen Erreger ausgerichtet ist, sondern die Identifikation aller für das definierte Krankheitsbild relevanten mikrobiellen Krankheitserreger erfaßt und zwar mit einer Wahrscheinlichkeit von 95%. Ferner soll das Verfahren den Nachweis der ursächlichen Mikroorganismen am Tag der Probennahme gewährleisten. Dem behandelnden Arzt sollten hierdurch ohne zeitliche Verzögerung wichtige therapierelevante Informationen in die Hand gegeben werden, was vor allem für eine Notfalldiagnostik bei fulminant verlaufenden Infektionskrankheiten von besonderem Interesse ist. In der gleichen Weise sollen Untersuchungen im Rahmen von Produktionsprozessen im Lebensmittel- und Pharmabereich ermöglicht werden und zwar im Sinne einer Produktions- und Qualitätskontrolle.

In der dargestellten Erfindung wird das Verfahren der Gesamtzellhybridisierung vorzugsweise in Reaktionsgefäßen, z. B. in Mikrotiterplatten, durchgeführt. Mikroorganismen, welche in biologischen Proben nachgewiesen werden sollen, werden hierbei auf dem Träger zur Nachweisreaktion immobilisiert. Diese neuartige Gesamtzellhybridisierung hat gegenüber der in situ Hybridisierung den Vorteil, daß die analysierbare Probenmenge um ein 10- bis 20-faches erhöht werden kann, d.h. insgesamt mehr Zielzellen der Untersuchung zugeführt werden, wodurch die Empfindlichkeit um mindestens eine Größenordnung gesteigert wird. Weiterhin fällt die zeitaufwendige mikroskopische Auswertung der Proben, die ein geschultes Personal notwendig macht, durch die einfache Handhabbarkeit der z.B. Mikrotiterplatten-Technik weg. Ein Auswechseln der Puffer kann ohne Zentrifugationsschritte und automatisiert erfolgen, da die Untersuchungsproben auf den Trägermaterialien immobilisiert sind. Die Auswertung kann anschließend in einem Mikrotiterplatten-Lesegerät erfolgen, das in jedem Labor vorhanden ist.

Das dargestellte Verfahren beschreibt außerdem eine einheitliche Fixierung und Permeabilisierung für jeweils die Gruppe der Gram-positiven und der Gram-negativen Bakterien und der Pilze. Sowohl die Fixierung, als auch Permeabilisierung der einzelnen Gruppen sind hierbei in Temperatur und Inkubationszeit aufeinander abgestimmt um eine einheitliche Verfahrensführung in mehreren Reaktionsgefäßen zu ermöglichen. Das Hybridisierungs- und Detektionsverfahren ist für alle Nachweisgruppen identisch. Zur Hybridisierung werden spezifische Sonden bereitgestellt, die an bestimmte Bereiche der Nukleinsäuren, insbesondere der ribosomalen RNA der nachzuweisenden Mikroorganismen binden. Der anschließende Nachweis der Hybridisierungsprodukte kann sowohl automatisiert mit Hilfe von bestimmten Lesegeräten wie z. B. ELISA-Readern (Hybridisierung in Reaktionsgefäßen), als auch visuell mit dem Mikroskop (Hybridisierung auf Objektträgern) erfolgen. Mit dem dargestellten Verfahren wird die Aufgabe gelöst, einen weitgehend vollständigen Nachweis, vorzugsweise jedoch mindestens 95% der schädlichen bzw. nützlichen Mikroorganismen in einer biologischen Probe zu erbringen. Hierdurch wird eine schnelle Diagnose und eine daraus resultierende spezifische Therapie z. B. bei Infektionen ermöglicht. Das Verfahren ist routinemäßig auch von ungeschultem Personal durchzuführen und aufgrund seiner einfachen Bestandteile sehr kostengünstig. Die Sensitivität geht bis auf Einzelzell-Ebene zurück, und es wird eine Spezifität von 100% verglichen mit dem klassischen Nachweisverfahren, der Kultivierung von Mikroorganismen erreicht.

Ein Gegenstand der Erfindung ist ein

Verfahren zur direkten Identifizierung von Mikroorganismen in einer biologischen Probe für die Diagnose von Sepsis, umfassend die Schritte:

(a) Aufteilen einer zu untersuchenden biologischen Probe in mehrere Teilproben,

(b) Vorbereiten der Teilproben für das Nachweisverfahren, wobei in der Probe vorhandene Mikroorganismen immobilisiert werden,

(c) Inkontaktbringen von mehreren der vorbereiteten Teilproben mit jeweils einer oder mehreren markierten Hybridisierungssonden, die einen Hybridisierungsbereich mit einer Länge bis 100 nt aufweisen, wobei

(i) jeweils unterschiedliche Hybridisierungssonden oder Kombinationen von Hybridisierungssonden für einzelne Teilproben verwendet werden,

(ii) die Hybridisierungssonden unter gleichen Hybridisierungsbedingungen angewendet werden,

(iii) die Hybridisierungssonden einen Hybridisierungsbereich enthalten, der zu Zielsequenzen komplementär ist, ermittelt aus Nukleinsäuren, die spezifisch für mögliche in der Probe vorhandene Mikroorganismen sind, wobei die Mikroorganismen für Sepsis verantwortlich sind, und

(iv) die Zielsequenzen so ermittelt werden, dass sie im Inneren der Zelle für eine Hybridisierung verfügbar sind, und

(d) Nachweisen von gebundenen Markierungen in den Teilproben,

wobei ein Satz von markierten Hybridisierungssonden verwendet wird, umfassend:

(I) gruppenspezifische Sonden, jede Sonde enthaltend eine der Sequenzen gemäß SEQ ID NO. 2, 3, 5, 6, 17 und 38 oder eine dazu mindestens 85 % identische Sequenz, und

(II) speziesspezifische Sonden, jede Sonde enthaltend eine der Sequenzen gemäß SEQ ID NO. 11, 12, 13, 14, 15, 16, 18, 19, 20, 21, 22, 23, 24, 25, 36 und 37 oder eine dazu mindestens 85 % identische Sequenz.

Das erfindungsgemäße Verfahren erlaubt die Identifizierung von Mikroorganismen, z.B. Bakterien, Pilzen, Protozoen und mehrzelligen Parasiten. Vorzugsweise wird das Verfahren zur Identifizierung von Bakterien oder/und Pilzen verwendet.

Die Ausgangsmaterialien, auf welche die vorliegende Erfindung angewendet werden kann, sind entweder biologische Proben, die direkt potentiell schädliche oder nützliche Mikroorganismen enthalten können oder mikrobielle Kulturen, die von solchen Proben angelegt worden sind. Hierbei handelt es sich besonders um klinische Proben, wie z. B. Sputum, bronchioalveoläre Lavagen, Blut, Wundabstriche, Biopsiematerialien aus unterschiedlichen Geweben, Liquores u.a. Hiervon sind jene Proben von besonderem Interesse, die von Patienten mit Sepsis stammen, bei welchen aufgrund des klinischen Bildes eine schnelle Diagnose nötig ist. Vor dem Überführen der biologischen Proben in geeignete Reaktionsgefäße, werden diese in der Regel in einem geeigneten Medium, z. B. einem Anreicherungs- oder Kulturmedium, mindestens jedoch einer physiologische Kochsalzlösung, suspendiert und gegebenenfalls verdünnt. Das Medium kann gegebenenfalls so gewählt werden, daß es eine Vermehrung der in der Probe vorhandenen Mikroorganismen erlaubt.

Das Verfahren ermöglicht einen direkten Nachweis von Mikroorganismen im Probenmaterial wobei der übliche Transport des Probenmaterials von der Entahme beim Arzt zum Untersuchungslabor berücksichtigt wird. In manchen Fällen, z.B. bei der Untersuchung von Blutproben, kann der Transport als Vorkultivierung genutzt werden. Eine derartige Vorkultur kann die Empfindlichkeit des Nachweisverfahrens verbessern. Im Gegensatz dazu wird bei herkömmlichen Verfahren der Keim erst nach dem Transport angezüchtet und anschließend isoliert, wodurch aufgrund der Kultivierungsdauer und der möglichen Auswahl suboptimaler bzw. falscher Medien ein großer Zeitverlust entsteht.

In manchen Fällen kann eine Voruntersuchung der Probe oder/und von Teilproben vor dem eigentlichen Nachweisverfahren erfolgen. Eine derartige Voruntersuchung, die ohne Kultivierung auskommt, kann in manchen Fällen integraler Bestandteil des Nachweisverfahrens sein. Beispielsweise kann die Voruntersuchung eine konventionelle Anfärbung, z.B. eine Gram-Färbung der Probe oder/und von Teilproben umfassen. Auf diese Weise kann die Anzahl von zu untersuchenden Teilproben erheblich reduziert werden.

Schritt (b) des Verfahrens beinhaltet das Vorbereiten der Teilproben für das Nachweisverfahren. Diese Vorbereitung beinhaltet ein Immobilisieren von Mikroorganismen, welches beispielsweise durch Trocknung, Filtration oder/und Zentrifugation erfolgen kann. Der Immobilisierungsschritt kann durch Verwendung vorbehandelter Träger oder Gefäße qualitativ oder/und quantitativ verbessert werden. Hierzu können beispielsweise Träger bzw. Gefäße eingesetzt werden, die mit Polylysin beschichtet sind.

Zur Konservierung der Zellstrukturen und insbesondere der Ribosomen und der ribosomalen RNA werden die Proben mit einem geeigneten Fixierungsmittel versetzt. Die Fixierung kann hierbei entweder vor oder nach dem Immobilisieren der Probe in einem Reaktionsgefäß oder auf einem Objektträger stattfinden. Die Fixierung kann als unterstützende Maßnahme zur Immobilisierung der Mikroorganismen dienen.

Es gibt bislang kein einheitliches Fixierungsprotokoll für Gram-positive und Gram-negative Bakterien bzw. Pilze (siehe hierzu (2)). Das verwendete Fixierungsmittel wird in der vorliegenden Erfindung daher entsprechend der Zellwandstruktur der jeweiligen Mikroorganismen gewählt. Für Gram-positive Bakterien und Pilze wird z. B. eine EtOH-PBS-Fixierung (1:1 Vol-%) bei Raumtemperatur oder eine Hitzefixierung für 10 min bei 80 °C durchgeführt. Es können aber auch andere Fixierungsmittel verwendet werden. Bei Gram-negativen Bakterien kann eine gepufferte Formalinlösung zur Fixierung verwendet, die ebenfalls bei Raumtemperatur inkubiert wird. Prinzipiell können alle Fixierungsmittel verwendet werden, die die vorhandenen Strukturen durch Denaturierung (s. Tabelle 1 A) oder/und Quervernetzung (s. Tabelle 1 B) konservieren.

Tabelle 1A: Denaturierende Fixierungsmittel: nach Haase (18)

| Name | Bedingungen |
|---|---|
| Ethanol-Essigsäure | Ethanol-Essigsäure, 3:1 15 min, EtOH 5 min, Raumtemperatur |
| Ethanol | Ethanol, 20 min, 4°C |
| Methanol-Aceton | Methanol-Aceton 1:2 (v/v) 4 min, -20°C; |
| Methanol | Methanol 20 min, -4°C |
| Aceton | AcetonAceton 4 min, -20°C; Aceton 20 min, 4 °C; |
| Chromsäure | Chromsäure, 0,1 %, 4°C, 20 min |
| Picrinsäure | 75%-Lösung, 2h, Raumtemperatur |
| Quecksilberchlorid | 5%, 2h, Raumtemperatur |
| Carnoy's Fixierung | Ethanol-Chloroform-Eisessig 60:30:10 (v/v/v) über Nacht bei Raumtemperatur; |

Tabelle 1b: Quervernetzende Fixierungsmittel: nach Haase (18)

| Name | Bedingungen |
|---|---|
| Formaldehyd | 0,1-4%, Raumtemperatur, 20 min |
| Glutaraldehyd | 0,1-2% Raumtemperatur, 20 min |
| Paraformaldehyd-Lysin-Periodat (PLP) | PLP, Raumtemperatur, 15 min |
| Ethyldimethylaminopropylcarbodiimid | 1 %, Raumtemperatur, 20 min |
| Dimethylsilserimidat | 20 mg/ml, 4°C, 30 min |

Nachdem die Bakterien in adäquater Weise in einem Reaktionsgefäß oder auf einem Objektträger immobilisiert (z. B. durch Filtration, Trocknung, Zentrifugation, Adhärenz an funktionelle Gruppen des Plastik- bzw. Glasträgers) und fixiert wurden, kann - sofern erforderlich - eine Permeabilisierung der Zellen erfolgen, um die freie Diffusion der Hybridisierungssonden in die Zelle zu ermöglichen. Die Permeabilisierung kann beispielsweise durch enzymatische, chemische, mechanische oder/und thermische Methoden erfolgen.

Wie bei der Fixierung gibt es bislang kein einheitliches Verfahren für die Permeabilisierung von Gram-positiven und Gram-negativen Bakterien, sowie Pilzen (2). Bislang wurde empirisch für jeden Gram-positiven Organismus ein auf diesen speziell abgestimmtes Protokoll verfolgt, das auf andere Gram-positive Organismen bzw. Pilze nicht in gleicher Form anwendbar war. In einer Veröffentlichung von Roller *et al.*, (36) werden z. B. verschiedene Gram-positive Bakterien nur einer bestimmten Fixierung unterworfen und nicht weiter permeabilisiert. In Veröffentlichungen von Matsuhisa *et al.* (33) und Krimmer *et al.*, (24) wird z.B. im Anschluß an die Fixierung verschiedener Gram-positiver Staphylococcus Spezies eine Permeabilisierung mit unterschiedlichen Enzymen angeschlossen. Ein Anliegen der vorliegenden Erfindung ist es daher, erstmalig ein einfaches Verfahren zu entwickeln, das auf die gesamte Gruppe der Gram-positiven Bakterien und die der Pilze gemeinsam anwendbar ist. Aufgrund ihrer weniger komplexen Zellwandstruktur ist die Permeabilisierung bei Gram-negativen Bakterien schon durch die vorhergehende Fixierung und eine einfache, aufsteigende Ethanolreihe gewährleistet (je 3 Minuten 50, 80, 96 % EtOH). Eine weitere Permeabilisierung wird nur dann nötig, wenn die Detektion der Sonden über enzymmarkierte oder indirekt detektierte Oligonukleotide erfolgt. In diesem Fall wird eine Behandlung mit Lysozym oder/und Proteinase K oder einem ähnlichen zellwandlytischen Enzym in einer Konzentration durchgeführt, welche gewährleistet, daß die Zellen zwar für die Detektionsagentien zugänglich sind, nicht aber, daß die Ribosomen nach außen bzw. Ribonukleasen nach innen diffundieren können. Die Behandlung wird z. B. mit 0,1 mg/ml Lysozym oder/und Proteinase K für 10 Minuten durchgeführt. Zusätzlich kann eine weitere thermische (z.B. Mikrowelle), chemische oder mechanische Behandlung ggf. nötig sein. Das Permeabilisierungsverfahren für die Gruppe der Gram-positiven Bakterien besteht aus einer simultanen Lysozym- und Lysostaphinbehandlung Die Lysozymkonzentration beträgt vorzugsweise 100 $\mu$g - 2 mg/ml, insbesondere etwa 1 mg/ml. Die Lysostaphinkonzentration beträgt vorzugsweise 0,1 bis 200 $\mu$g/ml, insbesondere 100 $\mu$l/ml. Zur Vereinfachung der Durchführung bzw. zur automatisierten Anwendbarkeit werden die dargestellten Permeabilisierungsverfahren jeweils für 15 Minuten bei 37°C durchgeführt.

Nachdem die Probenmaterialien in geeigneter Weise auf den Trägermaterialien immobilisiert, und die Zellen fixiert und permeabilisiert sind, müssen die Proben mit der Hybridisierungslösung in Kontakt gebracht werden. Gegebenenfalls kann vor der Hybridisierungsreaktion eine Vorbehandlung der Probe oder Prähybridisierung stattfinden. Bei dieser werden z. B. unspezifische Bindungen durch die Inkubation mit kleinen DNA-Fragmenten oder nicht-bindenden Oligonukleotiden blockiert bzw. endogene Peroxidasen durch $H_2O_2$ oder andere Chemikalien inhibiert.

Die Hybridisierungslösung enthält die Hybridisierungssonden in einer geeigneten Konzentration von z.B. 5 ng/$\mu$l in einer gepufferten Lösung sowie vorzugsweise die Stringenz beeinflussende Agentien wie Salze, Detergenzien, Formamid, DMSO etc. Die Zusammensetzung ist so gewählt, daß eine optimale Hybridisierungseffizienz mit einer ausreichender Spezifität verbunden ist. Besonders bevorzugt wird eine Hybridisierungslösung eingesetzt, die 0 bis 50% (vol/vol) eines organischen polaren Lösungsmittels, eine Salzkonzentration vom 0,5 bis 1,5 mol/l, 0 bis 0,1% (w/v) eines Detergenz und einen pH-Wert von 7 bis 8,5 aufweist. Der Hybridisierungspuffer besteht in der vorliegenden Erfindung z.B. aus einer Lösung aus 20% (v/v) Formamid, 0,9 M NaCl, 0,01% (w/v) SDS und 20 mM Tris-HCl pH 8,0. Gemäß dem Stand der Technik kann die Hybridisierung bei Temperaturen von 15 - 60° C und für 10 - 120 min durchgeführt werden (49), wobei die Bedingungen auf die jeweils verwendete Sonde abgestimmt werden, um eine möglichst hohe Spezifität und Hybridisierungseffizienz zu erzielen. In keinem der bislang veröffentlichten Verfahren wurden aus diesem Grund daher einheitliche Hybridisierungsbedingungen für unterschiedliche Sonden verwendet. Zur Vereinfachung der Durchführung und zur automatisierten Anwendbarkeit ist es daher bevorzugt ein Verfahren bereitzustellen, das einheitliche Stringenzbedingungen sowie Hybridisierungstemperaturen und -zeiten für alle verwendeten Sonden bzw. Sondenkombinationen erlaubt. Die Hybridisierung wird in Reaktionsgefäßen daher einheitlich vorzugsweise bei 37°C oder 46°C für z.B. 90 min durchgeführt. Zur Entfernung unspezifisch gebundener Sonden erfolgt nach der Hybridisierungsreaktion ein stringenter Waschschritt. Dazu muß die Stringenz des des Waschpuffers höher sein als die des Hybridisierungspuffers, sie darf aber nicht so hoch sein, daß sie die spezifische Bindung der Sonde an ihre Zielsequenz beeinträchtigt. Die höhere Stringenz wird z. B. durch eine niedrigere Salzkonzentration oder durch eine z.B. um 2°C erhöhte Waschtemperatur erreicht. Zur Vereinfachung der Anwendbarkeit ist daher bevorzugt ein Verfahren bereitzustellen, das einheitliche Hybridisierungspuffer sowie Hybridisierungstemperaturen und -zeiten für alle verwendeten Sonden bzw. Sondenkombinationen erlaubt. Nach dem Waschvorgang erfolgt die Detektion der hybridisierten Sonden entsprechend dem verwendeten Markierungsmolekül.

Für das erfindungsgemäße Verfahren werden in Schritt (c) Hybridisierungssonden verwendet, die gegen zugängliche Bereiche ribosomaler RNA intakter Ribosomen der nachzuweisenden Mikroorganismen gerichtet sind.

Zur Herstellung von geeigneten Hybridisierungssonden, die an spezifische Bereiche der ribosomalen RNA binden, wurden verschiedene Verfahren beschrieben (s. dazu Kohne US456729, Hogan *et al.*, WO88/03957, Lane *et al.*, WO90/15157 und andere). Grundlage dieser Verfahren sind Vergleiche von Nukleinsäure-Zielsequenzen öffentlich zugänglicher Datenbanken. Da die Zugänglichkeit von Nukleinsäure-Zielsequenzen und insbesondere ribosomaler RNA innerhalb ganzer intakter Zellen beschränkt ist (2), (13),(14), ist eine Reihe von Sonden, die bei den herkömmlichen Hybridisierungsverfahren an extrahierten und somit freiliegenden Nukleinsäuren eine ausreichend hohe Spezifität besitzen, für die Ganzzellhybridisierung nur eingeschränkt oder gar nicht verwendbar. Fuchs *et al.* untersuchten dieses Problem und kartierten systematisch die Zugänglichkeit von Bereichen auf der rRNA intakter Ribosomen von E. coli (14). Ergebnisse dieser Gruppe und eigene Erfahrungen zeigen jedoch deutlich, daß diese Bereiche nicht direkt auf andere Organismen übertragen werden können. Für die Entwicklung spezifischer Sonden bedeutet dieses daher, daß die Funktionalität der jeweiligen Sonden empirisch ausgetestet werden muß. Zunächst muß der Hybridisierungsbereich der Sonde ermittelt werden, der die beste Spezifität und Hybridisierungseffizienz hinsichtlich eines definierten Ziel-Mikroorganismus gewährleistet. Ist dieser Hybridisierungsbereich ermittelt, wird die Sondensequenz im Rahmen des Hybridisierungsbereichs so variiert, daß Hybridisierungstemperatur und -zeit sowie gegebenenfalls der Hybridisierungspuffer mit den anderen Sonden übereinstimmt. Schließlich wird jede entwickelte Sonde vorzugsweise an mindestens 5 Vertretern der eigentlichen Zielgruppe und an mindestens 30 Nicht-Zielgruppen Organismen ausgetestet. Außerdem werden Tests an klinischen Proben durchgeführt, um eventuelle Kreuzreaktionen mit weiteren in der Probe vorhandenen Mikroorganismen oder eukaryotischen Zellen auszuschließen. Mit dieser Methode können Hybridisierungssonden, die eine spezies-, gattungs- oder gruppenspezifische Erkennung einer Vielzahl von Mikroorganismen erlauben und zur Verwendung für das erfindungsgemäße Verfahren geeignet sind, identifiziert werden.

Ein Anliegen der vorliegenden Erfindung war es nicht nur einzelne, spezifische Sonden zu entwickeln, die in der Hybridisierung eingesetzt werden können, sondern vielmehr einen Satz von Sonden, der weitgehend, z.B. zu mindestens 95 % die relevanten Mikroorganismen, insbesondere Bakterien oder/und Pilze, abdeckt, die für ein bestimmtes Krankheitsbild bzw. einen typischen Befall verantwortlich sind. Die Angabe "relevant" umfaßt dabei sowohl schädliche (z.B. pathogene) als auch nützliche (z.B. im Sinne einer Qualitätskontrolle) Keime. Ein derartiger Satz von Hybridisierungssonden kann eine vollständige mikrobielle Diagnose, z.B. beim Auftreten einer bestimmten Infektionskrankheit erlauben. Unter "bestimmt" soll dabei der Umstand zum Ausdruck gebracht werden, daß ein Arzt bei seinem Patienten aufgrund seiner Kenntnisse das Vorliegen einer "bestimmten" Krankheit annimmt und zur genauen Befunderhebung ein diagnostisches Verfahren einleitet. Das diagnostische Fenster wird durch die vorliegenden Krankheitssymptome bestimmt. Innerhalb dieses Fensters erlaubt das vorliegende Verfahren eine vollständige bzw. zumindest weitgehend vollständige mikrobielle Diagnose. Weiterhin erlaubt das Verfahren auch eine weitgehende vollständige mikrobielle Diagnose von in einer Lebensmittelprobe vorhandenen produktionsrelevanten Mikroorganismen, wobei sich "produktionsrelevant" auf für die Qualität des Lebensmittels nützliche oder schädliche Keime, insbesondere Bakterien oder/und Pilze bezieht.

Ein derartig breites diagnostisches Spektrum ist trotz verschiedener Veröffentlichungen über spezifische Sonden für bestimmte Mikroorganismen bisher nicht dargestellt worden ((24); (11); (45)). Von einigen Arbeitsgruppen wurde dieses Problem daher durch die Verwendung einer umfassenden Sonde umgangen, mit welcher Bakterien generell abgedeckt sind (WO9100926; WO9015157, US5679520 und andere). Durch die Verwendung von Sonden für das Phylum Bacteria, früher als Eubakterien bezeichnet, sollte in verschiedenen Bereichen des Körpers eine bakterielle Infektion nachgewiesen werden können. Diese Vorgehensweise ermöglicht zwar den Nachweis von Bakterien als solchen, aber keine spezifische Identifizierung. Im Gegensatz dazu werden in vorliegender Anmeldung mit Hilfe von spezifischen Sonden Gattungen von Mikroorganismen bzw. individuelle individuelle Mikroorganismen identifiziert, mit welchen ein Spektrum von Mikroorganismen mit 95%iger Abdeckung eines bestimmten Krankheitsbildes bzw. Lebensmittelbefalls erstellt werden kann. Hierdurch kann eine sichere Diagnose und eine z. B. damit verbundene spezifische Antibiotikatherapie ermöglicht werden. Die Sonden sind vorzugsweise für eine vereinfachte Gesamtzellhybridisierung unter einheitlichen Bedingungen geeignet. Aufgrund der dargestellten Probleme, die mit der Entwicklung spezifischer Sonden für die Gesamtzellhybridisierung verbunden sind, war es daher überraschend, daß tatsächlich ein Spektrum an Oligonukleotidsonden für klinisch oder lebensmittelmikrobiologisch relevante Mikroorganismen entwickelt werden konnte, das den genannten Anforderungen standhält.

Neben dem Einzeleinsatz der hier ausgewiesenen Sonden, die zum Nachweis von bakteriellen Spezies (z. B. *Streptococcus pneumoniae, Pseudomonas aeruginosa* usw.) oder Gruppen von mehreren Spezies (z. B. Enterobacteriaceae) oder Gattungen (Staphylococcus, Streptococcus) oder Sonden zum Nachweis von Phyla (z. B. Bacteria) verwendet werden können, ist auch der kombinierte Einsatz der ausgewiesenen Sonden Teil der vorliegenden Erfindung. Dieser erfolgt dann, wenn durch den kombinierten Einsatz der Sonden eine klinisch relevante Fragestellung gelöst werden kann, wie z. B. der kombinierte Einsatz einer *Staphylococcus-Genus* Sonde zusammen mit einer *Staphylococcus aureus* Sonde zur Abklärung z. B. einer bakteriellen Infektion in einer Blutprobe mit *P.aeruginosa* oder die Identifizierung einer Gruppe mit einer definierten Antibiotikaresistenz bzw. Empfindlichkeit. Die verwendeten Sonden sind hierbei unterschiedlich markiert. Die Markierung kann entweder durch verschiedene Fluoreszenzfarbstoffe, im Falle eines direkten Nachweises bzw. durch verschiedene Enzyme oder Haptene im Falle eines indirekten Nachweises erfolgen.

Bedingt durch die umfangreichen genomischen Sequenzieraktivitäten, ist in den öffentlichen Datenbanken ein rapider Anstieg der Datensätze zu verzeichnen, der z.B. auch für die "small subunit" (SSU) rRNA-Datenbank zutrifft (47). Man muß einen erheblichen intellektuellen Aufwand betreiben, um aus der Datenflut die relevanten Informationen abzuleiten. Da die Datenbanken keiner Qualitätskontrolle unterliegen, steigt z.B. auch die Anzahl fehlerhafter Einträge. Andererseits werden die Daten eine zunehmend verfeinerte Differenzierung einzelner Mikroorganismen ermöglichen. Daher zeichnet es sich ab, daß für einige Gruppen von Mikroorganismen eine eindeutige Differenzierung zu nahe verwandten Gruppen nur mit zwei Sonden möglich ist. Nur wenn beide Sonden gleichzeitig binden, liegt die jeweilige Spezies vor.

Wie bereits ausgeführt eignet sich das erfindungsgemäße Verfahren zur Diagnostik von akuten, insbesondere fulminant verlaufenden Infektionskrankheiten wie Sepsis. Dabei wird eine Sondenkombination eingesetzt, die eine möglichst vollständige Identifizierung aller in einer bestimmten Probe potentiell vorkommenden mikrobiellen Krankheitserregern erlauben.

Die prozentuale Identität 1 zwischen 2 Hybridisierungsbereichen wird wie folgt bestimmt:

$$ I = \frac{n}{L} \times 100 $$

wobei n die Anzahl der unterschiedlichen Nukleotide in zwei Hybridisierungsbereichen ist und L die Länge des Basis-Hybridisierungsbereichs gemessen in Nukleotiden ist.

Bei der Diagnose einer Sepsis wird ein Satz von gruppenspezifischen Sonden für

(i) Prevotella und Bacteroides,
(ii) Enterobacteriaceae,
(iii) Staphylococcus;
(iv) Streptococcus, und
(v) Enterococcus,
und speziesspezifischen Sonden für
(i) Candida albicans,
(ii) Candida glabrata,
(iii) Candida krusei,
(iv) Candida parapsilosis,
(v) Enterococcus faecalis,
(vi) Klebsiella pneumoniae,
(vii) Pseudomonas aeruginosa,
(viii) Staphylococcus aureus,
(ix) Stenotrophomonas maltophilia,
(x) Streptococcus aglactiae,
(xi) Streptococcus pneumoniae und
(xii) Streptococcus pyogenes

verwendet.

Jede gruppenspezifische Sonden enthält einen Hybridisierungsbereich mit einer des Sequenzen gemäß SEQ ID NO. 2, 3, 5, 6, 17 und 38, oder eine dazu mindestens 85%, und insbesondere mindestens 90% identische Sequenz. Jede speziesspezifische Sonden enthält einen Hybridisierungsbereich mit einer der Sequenzen gemäß SEQ ID NO. 11, 12, 13, 14, 15, 16, 18, 19, 20, 21, 22, 23, 24, 25, 36 und 37 oder eine dazu mindestens 85%, insbesondere mindestens 90% identische Sequenz.

Neben gruppenspezfischen, d.h. gattungs-, familien-, subgenus- oder/und supergenus-spezifischen und speziesspezifischen Hybridisierungssonden können auch weitere Sonden eingesetzt werden, z.B. eine Phylum-spezifische Sonde, z.B. für Bakterien oder Pilze. Außerdem können Hybridisierungssonden eingesetzt werden, die spezifisch für eine mit Antibiotikumresistenzen assoziierte Nukleinsäuresequenz in Mikroorganismen sind, so daß neben einer Bestimmung von in der Probe vorhandenen Gattun-gen bzw. Spezies von Mikroorganismen gleichzeitig eine Antibiotikaresistenzbestimmung durchgeführt werden kann. Derartige mit Antibiotikumresistenzen assoziierte Nukleinsäuresequenzen finden sich beispielsweise auf ribosomalen Nukleinsäuresequenzen, z.B. auf der bakteriellen 23 S rRNA. Beispiele für geeignete Hybridisierungssonden, die insbesondere den Nachweis von Resistenzen gegen Macrolid-, Lincosamid-, Aminoglykosid-, Aminocyclitol-, Tetracyclin- und Chloramphenicol-Antibiotika erlauben, sind in PCT/EP99/0 35 27 offenbart.

Die zur Gesamtzellhybridisierung verwendeten Sonden enthalten Bereiche, die zu den Zielsequenzen komplementär sind (d.h. Hybridisierungebereiche). Diese Hybridisierungsbereiche können eine Länge bis 100 nt, vorzugsweise 17 bis 22 nt aufweisen. Sie sind so konzipiert, daß die entsprechende Zielgruppe an Organismen eine vorzugsweise 100%ig passende Sequenz aufweist, während der nächst verwandte Nicht-Zielgruppenorganismus eine um mindestens eine Base geringere Identität aufweist. Weiterhin binden die Sonden spezifisch an ihre Zielstrukturen, nicht aber an Nicht-Zielstrukturen trotz Verwendung einheitlicher Hybridisierungsbedingungen, und sie sind möglichst kurz, um die Diffusion durch die permeabilisierten Zellhüllen zu erleichtern. Sie besitzen eine hohe Spezifität, um in einem stringenten Hybridisierungspuffer einen Einbasenunterschied diskriminieren zu können.

Die Methoden für die Herstellung geeigneter einfach oder mehrfach markierter Nukleinsäuresonden sind Stand der Technik und in der Literatur mehrfach ausführlich beschrieben worden ((42), (23)). Die Sonden können z. B. mit Enzymen markiert sein oder, eingebunden in ein indirektes Detektionssystem, mit einem Molekül (z. B. Biotin, Digoxigenin oder einem anderen Hapten), das über ein zweites enzymmarkiertes System (z. B. Streptavidin, Anti-Digoxigenin oder Anti-Hapten) detektiert wird. Die bevorzugte Markierung der vorliegenden Erfindung erfolgt mit einem Enzym, insbesondere mit Peroxidase.

Die Detektion der verschiedenen Nachweissysteme wird entsprechend den Anforderungen der jeweiligen Markierung durchgeführt. Der Nachweis von Sonden, die an Fluoreszenzfarbstoffe gekoppelt sind, kann z. B. mikroskopisch mit verschiedenen Filterkombinationen erfolgen. Für die enzymmarkierten Sonden (direkt oder indirekt) erfolgt die Detektion des Signals mit chromogenen Substraten (Reaktionsgefäßhybridisierung), Chemolumineszenz oder präzipitierenden Farbstoffen (mikroskopischer Nachweis). Ein Nachweis von Peroxidase-gekoppelten Oligonukleotidsonden erfolgt bevorzugt z. B. mit den chromogenen Substraten 2,2'-Azino-bis(3-ethylbenzthiazolin-6-sulfonsäure) (ABTS), o-Phenylendiamin (OPD), 3,3'5,5'-Tetramethylbenzidin (TMB), o-Dianisidin, 5-Aminosalicylsäure (5AS) oder mit den für histologische Präparate geeigneten präzipitierenden Substraten wie 3,3'-Diaminobenzidin (DAB), die mit verschiedenen 2-wertigen Kationen (z.B. Kobalt) kombiniert werden können, 3-Amino-9-ethylcarbazol (AEC), 4-Chlor-1-naphthol (4C1 N), V-VIP (Vector-VIP) und anderen.

Ein Problem, das vor allem bei langsam wachsenden Mikroorganismen auftreten kann, ist, daß die Anzahl an ribosomaler RNA in den Zielzellen so gering ist, daß die kritische Schwelle (bei Fluorochromen z. B. von 10.000 Fluoreszenzmolekülen pro bakterielle Zelle) nicht überschritten wird. Zur Signalverstärkung können aber verschiedene Verfahren angewendet werden, die mit der Gesamtzellhybridisierung kompatibel sind. So können z.B. mehrere gegen ribosomale RNA gerichtete, markierte Oligonukleotidsonden, die eine gleiche oder zumindest eine ausreichende Spezifität für die entsprechenden Zielorganismen aufweisen, gleichzeitig verwendet werden. Außerdem können gegebenenfalls mehrere zur Verfügung stehende, eine ausreichende Spezifität aufweisende Sonden, die gegen die 5S rRNA, 16S rRNA und/oder 23S rRNA gerichtet sind, gleichzeitig verwendet werden.

Beim Einsatz von enzymmarkierten Sonden können zur Ermittlung der Gesamtzahl der Mikroorganismen Phyla-spezifische Sonden (z. B. gegen Bacteria) in Kombination mit einer speziesspezifischen Sonde verwendet werden. Die unterschiedlichen Sonden können hierbei jeweils mit zwei verschiedenen Enzymen markiert sein. Die Phyla-spezifische Sonde könnte z.B. an das Enzym Meerrettich-Peroxidase und die speziesspezifische Sonde an das Enzym Alkalische Phosphatase gekoppelt sein. Beide Sonden können aber auch hintereinander, gegebenenfalls jedoch auch gleichzeitig hybridisiert werden. Jedes Enzym ist anschließend für einen spezifischen Farb- oder Fluoreszenzniederschlag verantwortlich, der photometrisch oder/und mikroskopisch bestimmt werden kann. Bei Verwendung nur eines Enzyms, z.B. der Meerrettich-Peroxidase, muß nach jeder Hybridisierungs- und Farbreaktion eine Inaktivierung , z.B. Hitzeinaktivierung des Proteins stattfinden, bevor eine weitere Hybridisierung mit Detektion angeschlossen werden kann. Die Summe der Substratumsetzungen kann anschließend wieder photometrisch oder/und mikroskopisch ausgewertet werden.

Das hier ausgewiesene Verfahren kann zur Anwendung auf Objektträgern auch für einen kombinierten Einsatz einer unspezifischen Nukleinsäurefärbung (z. B. DAPI, Propidiumiodid) zusammen mit einer fluoreszenzmarkierten Phyla-spezifischen Sonde und einer weiteren, fluoreszenzmarkierten Sonde (gattungs-, gruppen-oderspeziesspezifischen Sonde) verwendet werden. Mit Hilfe z. B. einer DAPI-Färbung kann die diagnostische Sicherheit erhöht werden, indem spezifisch fluoreszierende Zellen von unspezifisch fluoreszierenden Partikeln oder Autofluoreszenz unterschieden werden können. Mit Hilfe z. B. einer DAPI-Färbung, einer Phyla-spezifischen Sonde und einer speziesspezifischen Sonde kann der Anteil bestimmter zu identifizierender Mikroorganismen in einer Infektion festgestellt werden bzw, ob es sich um eine Mono- oder eine Mischinfektion handelt. Die unterschiedlichen Zahlen, die mit den verschiedenen Färbungsmethoden, bzw. Sonden gewonnen werden, entsprechen hierbei der Gesamtzahl der Organismen (DAPI-Färbung) im Verhältnis zu der Gesamtzahl an Organismen, die für die Sonden zugänglich sind, bzw. eine zum Nachweis ausreichende Menge an Ribosomen aufweisen im Verhältnis zur Anzahl spezifisch nachgewiesener Organismen.

Enzyme können auch zur Signalverstärkung verwendet werden. Hierfür können, wie bereits beschriebenen, direkt enzymmarkierte Oligonukleotidsonden oder indirekte Nachweisverfahren verwendet werden. Mit Hilfe der Enzymaktivität wird dann eine Vielzahl bestimmter Substrate gespalten, deren Produkte eine Signalverstärkung bewirken. Beispielsweise kann durch das TSA System (DuPont, NEN Research Products, Boston, MA) eine fünfbis elffache Amplifikation des Signals erzielt werden (40), (3). Das Enzym Meerettich-Peroxidase, das entweder direkt an die jeweilige Sonde gekoppelt ist oder in einem indirekten System verwendet wird, spaltet hierbei das Molekül Tyramid, wodurch ein fluoreszierender Niederschlag entsteht. Ein anderes System, das außerdem der Vermeidung von Hintergrundfluoreszenz der Zellen dient, ist die zeitverzögerte Fluoreszenz. Hierfür werden Chelate bestimmter Seltenerdmetall-Ionen wie z.B. Lanthaniden verwendet, die an organische Moleküle gekoppelt sind. Lanthaniden besitzen eine langanhaltende Lumineszenz, die mit einer Verzögerung von mehren Millisekunden im Vergleich zu Nanosekunden, die bei einer Hintergrundfluoreszenz auftreten (s. dazu US4150295 und US4058732) gemessen werden kann. Nach Angaben des Herstellers (DELFIA System von Wallac Oy, Finnland) geht die Sensitivität der Lanthaniden-Lumineszenz bis auf $5 \times 10^{-14}$ Mol/l zurück. Im Falle einer Detektion von ribosomaler RNA wie in der vorliegenden Erfindung, läge die Nachweisgrenze somit bei RNA wie in der vorliegenden Erfindung, läge die Nachweisgrenze somit bei 5 bis 50 Zellen/100 μl, wenn von einer Ribosomenzahl von $10^4$ bis $10^5$ Ribosomen pro Zelle ausgegangen wird (8). Bei langsam wachsenden Mikroorganismen ist jedoch von einer geringeren Anzahl auszugehen. Das System der Lanthaniden-Lumineszenz kann in diesem Fall mit Hilfe bestimmter Enzyme, z. B. der Meerrettich-Peroxidase, weiter verstärkt werden (WO9108490). Das Detektionslimit liegt hierbei bei $2 \times 10^{-16}$ Mol/l, was einer Keimzahl von 10 Zellen/100 μl entspricht, wenn bei langsam wachsenden Organismen von einer Ribosomenzahl von $5 \times 10^3$ ausgegangen wird (vgl. dazu US5663049).

Zur Erhöhung der Ribosomenzahl und damit auch der Sensitivität des Verfahrens kann in jedem Fall natürlich auch eine klassische Vorkultur der Mikroorganismen oder eine selektive Kultur durchgeführt werden. Dieser zusätzliche Schritt wird jedoch weitgehend vermieden.

Zur Erhöhung der Sensitivität kann auch ein kombiniertes Verfahren aus Gesamtzellhybridisierung und ELISA durchgeführt werden. Hierzu werden mit spezifischen Antikörpern, welche Oberflächendeterminanten des jeweiligen Mikroorganismus erkennen, beschichtete Reaktionsgefäße zur Immobilisierung verwendet und spezifische Oligonukleotidsonden für den anschließenden Nachweis. Es können jedoch auch spezifische Antikörper gegen bakterielle Toxine in Kombination mit enzymgekoppelten Oligonukleotidsonden verwendet werden. In diesem Fall wird ein klassischer Immunnachweis *in situ* mit der Gesamtzellhybridisierung gekoppelt. Wie bereits im vorangegangenen Teil werden hierbei für die anschließende Nachweisreaktion zwei verschiedene Enzyme, z. B. Meerrettich-Peroxidase und Alkalische Phosphatase verwendet, so daß ein jeweils unterschiedlicher Farb- oder Fluoreszenzniederschlag entsteht.

Ein weiterer Gegenstand der Erfindung ist die Verwendung eines Hybridisierungsverfahrens zur Identifizierung von Mikroorganismen in bestimmten biologischen Proben vorzugsweise aus verschiedenen Quellen, wobei jeweils verschiedene Teilproben vorzugsweise parallel mit unterschiedlichen Hybridisierungssonden behandelt werden, wobei die Hybridisierungssonden für Gruppen oder/und Spezies von der Probe vorhandenen relevanten Mikroorganismen spezifisch sind. Die Bezeichnung "aus verschiedenen Quellen" bedeutet, daß z.B. klinische Proben aus verschiedenen Patienten verwendet werden können. Das Verfahren kann in Reaktionsgefäßen, z.B. in Mikrotiterplatten, durchgeführt und gegebenenfalls automatisiert werden.

Noch ein weiterer Gegenstand der Erfindung ist ein Reagenzienkit zur Identifizierung von Mikroorganismen in biologischen Proben für die Diagnose von Sepsis umfassend einen Satz von markierten Hybridisierungssonden, die einen Hybridisierungsbereich mit einer Länge bis 100 nt aufweisen, wobei die Hybridisierungssonden unter gleichen Hybridisierungsbedingungen angewendet werden, und jeweils unterschiedliche Hybridisierungssonden einen Hybridisierungsbereich enthalten, der zu Zielsequenzen komplementär ist, ermittelt aus Nukleinsäuren, die spezifisch für mögliche in der Probe vorhandene Mikroorganismen verantwortlich für Sepsis sind und wobei die Sonden so ausgewählt sind, dass sie im Inneren der Zelle für eine Hybridisierung verfügbar sind, wobei der Satz von markierten Hybridisierungssonden umfasst:

(I) gruppenspezifische Sonden, jede Sonde enthaltend eine der Sequenzen gemäß SEQ ID NO. 2, 3, 5, 6, 17 und 38 oder eine dazu mindestens 85 % identische Sequenz, und

(II) speziesspezifsche Sonden, jede Sonde enthaltend eine der Sequenzen gemäß SEQ ID NO. 11, 12, 13, 14, 15, 16, 18, 19, 20, 21, 22, 23, 24, 25, 36 und 37 oder eine dazu mindestens 85 % identische Sequenz.

Weiterhin umfaßt der Reagenzienkit vorzugsweise Mittel zum Fixieren und Permeabilisieren von mikrobiellen biologischen Probenbestandteilen. Außerdem kann der Reagenzienkit Medien zum Aufbewahren von Proben sowie Hybridisierungs- und Waschlösungen jeweils in Form von Einzelbestandteilen oder in bereits vorgefertigter Form enthalten. Der Reagenzienkit ist insbesondere zur Verwendung für das erfindungsgemäße Verfahren vorgesehen.

Die Kits können für das dargestellte Verfahren zur Verwendung in Reaktionsgefäßen wie z. B. Mikrotiterplatten aber auch auf Objektträgern bereitgestellt werden. Je nach Anwendungsbereich enthalten die Kits Oligonukleotidsonden und Reagenzien in einer Zusammensetzung, die eine Nachweisreaktion im Sinne der Erfindung ermöglichen. Die Oligonukleotidsonden sind hierbei je nach Anwendungsbereich markiert, beispielweise mit Fluoreszenzfarbstoffen oder mit Enzymen. Bei der Verwendung von indirekten Nachweismethoden können die Sonden auch mit Haptenen wie z.B. Digoxigenin oder aber auch mit Biotin versehen sein.

Im Folgenden wird ein exemplarischer Kit dargestellt, der zum Nachweis von sepsisverursachenden Mikroorganismen verwendet werden kann. Sepsis bezeichnet ein schweres, lebensbedrohliches Krankheitsbild, in dem auf der (Schleim-)Haut oder in der Umwelt vorkommende Mikroorganismen in die Blutbahn eindringen. In einer Vielzahl der Fälle ist ein einziger Erreger Ursache der Infektion (ca. 95 %), und polymikrobielle Infektionen treten nur in ca. 5 % auf (37). Das Krankheitsbild ist hauptsächlich auf die Freisetzung von Toxinen wie z. B. das Toxin TSST-19 von *Staphylococcus aureus,* pyrogene Toxine von *Streptococcus pyogenes,* SLT von E. coli und Toxin A von *Pseudomonas aeruginosa,* sowie Zellwandbestandteilen wie z. B. Endotoxin und Teichonsäuren zurückzuführen. Falls die Infektion nicht frühzeitig erkannt und behandelt wird, kann die Menge der Bakterien im Blut so anwachsen, daß Bakterienfragmente wie z. B. LPS oder toxische bakterielle Zellwandkomponenten eine so massive Immunantwort auslösen, daß es zu einem septischen Schock und damit einhergehend zum Tod kommen kann. Die Mortalitätsrate der Sepsis ist von verschiedenen Faktoren abhängig und bewegt sich in einem Bereich von 30 - 70 %. Entscheidend für das Überleben der betreffenden Patienten ist daher ein schneller Nachweis der Infektionsverursacher und eine damit verbundene frühzeitige Behandlung. Das klassische Verfahren zur Identifikation der ursächlichen Bakterien einer Sepsis ist die Blutkulturdiagnostik, bei welcher eine Trübung, Hämolyse, Gasentwicklung oder das Redoxpotential angezeigt wird, mit anschließendem Anlegen von Subkulturen zur mikrobiellen Diagnostik. Trotz relativ hoher Sensitivität (ca. 99 % aller Erreger können mit drei Blutkulturen zu unterschiedlichen Zeitpunkten identifiziert werden), ist die klassische Diagnostik sehr langsam, da sie ein definitives Ergebnis erst nach 24 bis 48 Stunden nach dem positiven Auftreten einer Blutkultur liefern kann. Im Gegensatz hierzu wird im Folgenden ein Kit dargestellt, der die Erreger der Sepsis bereits nach ca. 3 Stunden nach einer positiven Blutkultur mit einer Effizienz von 95 % identifizieren kann, wodurch ein entscheidender Zeitgewinn von 26 bis 48 Stunden erzielt wird.

In einer bevorzugten Ausführungsform besteht der Kit aus einem Basiskit, der für alle Anwendungen gleich ist. Dieser enthält als Positivkontrolle eine markierte Phyla-spezifische Bacteria-Sonde, z.B. EUB338, welche zu einer Region auf der 16S rRNA komplementär ist, die in allen untersuchten Bakterien identisch ist (1), sowie eine markierte Phyla-spezifische Fungus-Sonde, z.B. PF2 (38) und eine Negativkontrolle, z.B. NonEUB338; (1). Weiterhin kann der Basiskit Reagenzien, Salze und/oder Puffer enthalten, die für eine optimale Fixierung, Permeabilisierung, Prähybridisierung bzw. Hybridisierung notwendig sind. Alle Reagenzien, die für den Hybridisierungsnachweis erforderlich sind, wie z.B. Streptavidin oder spezifische Antikörper im Falle eines indirekten Nachweises, können ebenfalls vorhanden sein. Der spezifische, zum Nachweis von Sepsis entwickelte Kit enthält zusätzlich eine oder mehrere gattungspezifische Sonden (z. B. Entero für Enterobacteriaceae oder Staphy für Staphylococcaceae), welche zu einer Region auf der 16S oder/und 23S Staphylococcaceae), welche zu einer Region auf der 16S oder/und 23S rRNA komplementär sind, die für potentiell in der Probe vorhandene pathogenen Keime spezifische, aber über einen Genus pathogener Keime konserviert sind, sowie eine oder mehrere speziesspezifische Sonden, welche gleichfalls zu einer Region auf der 16S oder/und 23S rRNA komplementär sind, die für einzelne Spezies der potentiell in der Probe vorhandenen pathogenen Keime spezifisch ist. Ein Beispiel für eine konkrete Sondenzusammensetzung ist in Tabelle 4 dargestellt.

Eine zusätzliche Anwendungsmöglichkeit des dargestellten Kits besteht in der Identifizierung des Resistenzstatus der nachgewiesenen Mikroorganismen. Hierzu werden zusätzlich eine oder mehrere Sonden in den Kit aufgenommen, die spezifisch gegen ein oder mehrere Gene oder RNA-Abschnitte gerichtet sind, mit welchen ursächlich Resistenzen gegen Antibiotika nachgewiesen werden können (vgl. hierzu PCT/EP99/03527).

Schließlich betrifft die Erfindung auch die
Verwendung eines Satzes von Oligonukleotiden, die einen Hybridisierungsbereich zur Hybridisierung von mikrobiellen Zielsequenzen enthalten, wobei der Hybridisierungsbereich eine Länge bis 100 nt aufweist und wobei der Hybridisierungsbereich jedes Oligonukleotids eine der Sequenzen gemäß SEQ ID NO. 2, 3, 5, 6, 11, 12, 13, 14, 15, 16, 17. 18, 19, 20, 21, 22, 23, 24, 25, 36, 37 und 38 oder eine dazu mindestens 85 % identische Sequenz enthält, im erfindungsgemäßen Verfahren.

Vorzugsweise tragen die Oligonukleotide eine oder mehrere Markierungsgruppen.

**Beispiele:**

Die vorliegende Erfindung soll durch die folgenden Beispiele illustriert werden, soll aber auf keinen Fall auf diese beschränkt bleiben.

Beispiel 1 :

Entwicklung eines einheitlichen Permeabilisierungs-/Hybridisierungsverfahrens für Gram-positive Bakterien

Im Gegensatz zur relativ einheitlichen Zellhülle von Gram-negativen Bakterien, ist die Peptidoglycanhülle Gram-positiver Bakterien so variabel, daß ihre chemische Struktur sogar zur taxonomischen Einteilung Gram-positiver Bakterien verwendet wird (39). Während die meist einschichtige Peptidoglycanhülle von Gram-negativen Bakterien in der Regel ohne weitere Permeabilisierungsmaßnahmen für fluoreszenzmarkierte Oligonukleotide zugänglich ist, stellen die vielschichtigen Zellhüllen von Gram-positiven Bakterien, die zudem einen hohen Vernetzungsgrad aufweisen, oft eine bedeutende Diffusionbarriere für Nukleinsäuresonden dar (2). Im Folgenden sollte zur Vereinfachung des Verfahrens daher ein einheitliches Permeabilisierungsverfahren entwickelt werden, mit welchem die unterschiedlichen Zellhüllen verschiedenster Gram-positiver Bakterien derart aufgeschlossen werden können, daß eine Ganzzellhybridisierung anschließend problemlos durchgeführt werden kann. Idealerweise sollte das Permeabilisierungsverfahren gleichzeitig mit der Hybridisierungsreaktion erfolgen.

Zur Etablierung eines einheitlichen Verfahrens wurden verschiedene Gram-positive Bakterien (*Staphylococcus aureus, Staphylococcus epidermidis, Streptococcus pyogenes, Clostridium difficile, Enteroccocus faecalis, Bifidobacterium bifidum)* durch Zugabe von einem Volumenanteil 96%igem EtOH fixiert. Anschließend wurden die Bakterien einmal mit PBS gewaschen und bei - 20 °C in einer 1:1 1 Mischung aus PBS und 96%igem EtOH zum Kulturmedium fixiert. Zur anschließenden Gesamtzellhybridisierung mit fluoreszenzmarkierten Sonden wurden je 10 μl der Probe auf einen teflonbeschichteten Objektträger mit 6 Aussparungen getropft und luftgetrocknet. Nach einer aufsteigenden EtOH-Reihe (je 3 min 50, 80 und 96 % EtOH) wurden die getrockneten Bakterien verschiedenen Permeabilisierungslösungen mit unterschiedlichen Enzymkonzentrationen ausgesetzt und anschließend bzw. gleichzeitig einer Ganzzellhybridisierung unterzogen.

Um eine gleichmäßige enzymatische Aktivität der verschiedenen Enzymlösungen zu gewährleisten, wurden die Zellen 5 min in den jeweiligen Permeabilisierungspuffer ohne Enzyme inkubiert. Nachdem die Proben vollständig benetzt waren, wurde eine Lösung aus zellwandlytischen Enzymen und Detergentien in verschiedenen Konzentrationen zugegeben. Während die Lysozymkonzentration mit 1 mg/ml konstant gehalten wurde, wurde die Lysostaphinkonzentration in einem Bereich von 0,1 - 100μg/ml variiert. Nachdem eine ideale Lysozym-Lysostaphinlösung gefunden wurde, wurden zur weiteren Optimierung verschiedene Detergentien in Kombination mit der Enzymlösung ausgetestet. Die anschließende Enzymreaktion wurde für 10 min bei 37 °C durchgeführt. Dann wurde eine Hybridisierung mit einer Bacteria-spezifischen Sonde EUB338 (GCTGCCTCCCGT AGGAGT; SEQ ID NO. 1) durchgeführt. Anschließend wurde mit Hilfe einer DAPI-Färbung, welche Nukleinsäure unspezifisch sichtbar macht, die Gesamtanzahl der Zellen ermittelt. Aus dem Verhältnis der DAPI gefärbten Zellen (Gesamtzahl)/Eub-gefärbte Zellen (für In Situ Hybridisierung zugänglich) wurde die Güte des Aufschlusses bestimmt. Für jede Bakterienart und Aufschlußbedingung wurden jeweils 5 zufällig ausgewählte Gesichtsfelder ausgezählt und die Ergebnisse ausgewertet. Die Resultate dieser Untersuchung sind in Tabelle 2 dargestellt.

Tabelle 2: Prozentualer Aufschluß Gram-positiver Bakterien bei verschiedenen Lysostaphinkonzentrationen

|  | 100 μg/ml Lysostaphin | 10 μg/ml | 1 μg/ml | 0,1 μg/ml |
|---|---|---|---|---|
| S.aureus | 22,5 % | 80,5% | 22,5% | 26% |
| S.epidermidis | 60% | 21,5% | 12% | 15,5% |
| S.pyogenes | 91 % | 100% | 100% | 100% |
| b.bifidum | 91 % | 100% | 60% | 55% |
| C.difficile | 82,5% | 63% | 51 % | 50% |
| E.faecalis | 100% | 100% | 100% | 100% |

Mit Hilfe der durchgeführten Versuche konnte zum ersten Mal ein kombiniertes Aufschluß- und Fixierungsprotokoll für Gram positive Bakterien erstellt werden. Optimal ist eine EtOH-Fixierung in Kombination mit einem Aufschluß der folgendermaßen aussieht. Zunächst wird die Probe 5 min in einem Präpermeabilisierungspuffer, der dem Hybridisierungspuffer enspricht, inkubiert. Anschließend werden 1 mg/ml Lysozym und 10 μg/ml Lysostaphin zugegeben und die Lösung für 10 min bei 37 °C inkubiert. Die Enzymreaktion wird durch eine 10 minütige Erhitzung auf 80 °C abgestoppt und anschließend wird die Hybridisierung wie oben beschrieben durchgeführt.

Das dargestellte Verfahren verwendet im Vergleich zu anderen publizierten Protokollen kostengünstige Standardreagenzien, die sich gut für Routineanwendungen eignen.

Beispiel 2:

Entwicklung und Austestung von Oligonukleotidsonden, die für die Ganzzellhybridisierung pathogener und protektiver Mikroorganismen in biologischen Proben geeignet sind

Zur Identifizierung von spezifischen Regionen auf der ribosomalen RNA, die sich aufgrund ihrer Primärstruktur für die Identifikation von Mikroorganismen eignen, wurde zunächst ein Computer-gestützter Vergleich der ribosomalen RNA Moleküle aller Zielorganismen und nahe verwandter Nicht-Zielorganismen durchgeführt. Anhand dieser Ergebnisse wurde anschließend empirisch ein spezifisches rRNA-Zugänglichkeitsprofil für den jeweiligen Organismus mit Hilfe fluoreszenzmarkierter Sonden erstellt. Aufgrund der Informationen der geeigneten Primärsequenzen und des Zugänglichkeitsprofils wurden schließlich gezielt Sonden, die für die Ganzzellhybridisierung geeignet sind, entwickelt. Diese Sonden wurden dann an mindestens 5 verschiedenen Zielorganismen und nahe verwandten Nicht-Zielorganismen ausgetestet. Zur Austestung von Zielorganismen mit speziesspezifischen Sonden wurden z. B. der Typstamm und vier weitere Stämme der entsprechenden Art verwendet, die aus klinischen Materialien isoliert wurden. Außerdem wurden möglichst alle bei einem bestimmten Krankheitsbild auftretenden Bakterienspezies auf die gleiche Weise untersucht. Die Sonden wurden während dieser Testphase jeweils so verändert, daß sie unter identischen Hybridisierungsbedingungen an die rRNA aller Zielorganismen, nicht aber an die rRNA irgendeines der Nicht-Zielorganismus binden.

Die auf diese Weise entwickelten Sonden können zur schnellen und sicheren Identifikation von Mikroorganismen bei definierten Krankheitsbildern oder in der Lebensmittelmikrobiologie eingesetzt werden. Hierbei wird darauf geachtet, daß mindestens 95% der bei einem definierten Krankheitsbild auftretenden Mikroorganismen von dem jeweiligen Sondensatz erfaßt werden. Weiterhin sollen mit Hilfe des dargestellten Verfahrens der Ganzzellhybridisierung z. B. klinisch relevante Fragestellungen beantwortet werden, wie z. B. die Identifikation von Mikrorganismen, die für lebensbedrohliche Infektionen wie toxisches Sepsis verantwortlich sind. Der Sondensatz ist in Tabelle 4 dargestellt.

Tabelle 4: Sondensatz für die mikrobielle Diagnose bei Sepsis:

| Namen | Sequenz (5'-3') | Spezifität | Häufigkeit (a) | Referenz/ Kommentar |
|---|---|---|---|---|
| gruppenspezifische Sonden | | | | |
| Bac303 SEQ ID NO. 2 | CCAATGTGGGGGACCTT | *Prevotella + Bacteroides* | 1,2 % | (32) |
| Entero SEQ ID NO. 3 | CCCCCWCTTTGGTCTTGC | Enterobacteria ceae (b) | 31,8 % | |
| Staphy SEQ ID NO. 5 | TCCTCCATATCTCTGCGC | Staphylococcaceae | 30,3 % | |
| Strept SEQ ID NO. 6 | CACTCTCCCCTTCTGCAC | Streptococcaceae | 10,6 % | |
| Entcoc SEQ ID NO. 17 | CCCTCTGATGGGTAGGTT | Zusammen mit Entfae eingesetzt, geeignet zur gattungsspezifischen Detektion von Enterokokken | 5,5 % (nicht-hämolysierende Streptokokken) | (34) |
| speziesspezifische Sonden | | | | |
| Canalb SEQ ID NO. 18 | GCCAAGGCTTATACTCGCT | *Candida albicans* | 1,9 % (Sproßpilze allgemein) | |
| Cangla SEQ ID NO. 19 | CCGCCAAGCCACAAGGACT | *Candida glabrata* | | |
| Cancru SEQ ID NO. 20 | GATTCTCGGCCCCATGGG | *Candida krusei* | | |
| Canpar SEQ ID NO. 21 | CCTGGTTCGCCAAAAAGGC | *Candida parapsilosis* | | |
| Entfae SEQ ID NO. 22 | CCCCTTCTGATGGGCAGG | Zusammen mit Entcoc eingesetzt geeignet zur gattungsspezifischen Detektion von Enterokokken | 5,5 % (nicht hämolysierende Streptokokken) | (34) |

(fortgesetzt)

| speziesspezifische Sonden | | | | |
|---|---|---|---|---|
| Klepne SEQ ID NO. 23 | CCTACACACCAGCGTGCC | *Klebsiella pneumoniae* | 5,9 % | |
| Fortsetzung Tabelle 4 | | | | |
| PseaerA SEQ ID NO. 11 | GCTGAATCCAGGAGCAAGC | *Pseudomonas aeruginosa* | 4.8 % | Zusammen mit PseaerB einsetzen |
| PseaerB SEQ ID NO. 12 | GGTAACCGTCCCCCTTGC | *Pseudomonas aeruginosa* | 4,8 % | Zusammen mit PseaerA einsetzen |
| Staaur SEQ ID NO. 13 | GAAGCAAGCTTCTCGTCCG | *Staphylococcus aureus* | 19,9 % | |
| Stema SEQ ID NO. 24 | GTCGTCCAGTATCCACTGT | *Stenotrophomonas maltophilia* | Keine Angabe | |
| Straga SEQ ID NO. 14 | GTAAACACCAAACMTCAGCG | *Streptococcus agalactiae* | 2 % (β. hämolysierende Streptokokken) | |
| Strpne SEQ ID NO. 25 | GTGATGCAAGTGCACCTT | *Streptococcus pneumoniae* | 2,5 % | |
| StrpyoA SEQ ID NO. 15 | CTAACATGCGTTAGTCTCTC | *streptococcus pyogenes* | 2. % (β-hämolysierende Streptokokken) | Zusammen mit StrpyoB einsetzen |
| StrpyoB SEQ ID NO. 16 | TCCAAAGCGTACATTGGTT | *Streptococcus pyogenes* | 2 % (β-hämolysierende Streptokokken) | Zusammen mit StrpyoA einsetzen |
| (a) Prozentuale Keimverteilung der Erreger einer Sepsis nach Rosenthal (b) alle *Enterobacteriaceae* mit Außnahme der Gattung *Proteus* | | | | |

Die ausgewiesenen Sonden werden in Kombination für bestimmte Fragestellungen angewendet . Die Sonden wurden für diese Anwendung so entwickelt, daß sie in einer Hybridisierungsreaktion unter gleichen Hybridisierungsbedingungen verwendet werden können.

Beispiel 3:

Detektion von Bakterien mittels Ganzzellhybridisierung in Proben von Patienten mit Sepsis

Die Sepsis ist ein lebensbedrohliches Krankheitsbild, bei welchem das Vorkommen von Bakterien im Blut mit schweren klinischen Symptomen, wie Temperaturen von über 38 °C, Schüttelfrost, Tachykardie, Hypotonie, Leukocytose mit Linksverschiebung, Anämie, Thrombozytopenie und pathologischer Serumelektrophorese einhergeht. Zum Nachweis von Sepsis werden Blutkulturen der betreffenden Patienten angelegt, von welchen mindestens zwei positiv sein sollten (6). Die Mortalität dieser Infektionskranheit liegt trotz des Einsatzes moderner Antibiotika zwischen 30 und 70% und ist von Faktoren, wie Immunstatus des Patienten, Art des Erregers oder Epidemiologie der Infektion (ambulant versus nosocomial) abhängig. Die herkömmliche mikrobielle Diagnostik basiert auf der Blutkultur. Da die Keimzahlen z. T. mit bisweilen unter 10 KBE/ml sehr niedrig sind, sollten beim Erwachsenen mehrmals im Abstand von 4 - 6 Stunden (mindestens zwei Mal) etwa 20 ml Blut entnommen und in ein Blutkultursystem eingebracht werden. Bei dem für diese Studie verwendeten Blutkultursystem (BacT/Alert®, BACTEC 9240®) wird die $CO_2$-Produktion der eventuell vorhandenen Mikroorganismen kontinuierlich gemessen. Bei der Überschreitung eines bestimmten Schwellenwertes kommt es dann zu einer automatischen Meldung. Im Anschluß daran wird eine Gram-Färbung durchgeführt und aerobe und anaerobe Subkulturen angelegt. Eine definitive mikrobielle Diagnostik der in den Blutkulturen gewachsenen Bakterien erfolgt schließlich erst 24 - 48 Stunden nachdem die Blutkulturen positiv waren. Aufgrund dieser langen Zeitspanne ist eine schnelle Identifikation der ursächlichen Mikroorganismen und eine damit verbundene rasche, erregerspezifische Therapie nicht möglich. Eine frühzeitige, spezifische Antibiotikatherapie wäre aber wünschenswert, da diese eine Reihe von Vorteilen gegenüber der breiten, unspezifischen Initialtherapie aufweist. So ist beispielsweise das spezifisch gegen Streptokokken und Pneumokokken verwendete Penicillin G nicht nur billiger als das Breitbandantibiotikum Vancomycin, sondern auch 10fach aktiver (26). Weiterhin wird durch einen möglichst kurzen Einsatz spezifischer Antibiotika einer möglichen Restistenzentwicklung entgegengewirkt, welche fatale Folgen hätte, da ein Versagen der Initialtherapie fast unweigerlich den Tod des betroffenen Patienten nach sich zieht.

Zum schnellen und spezifischen Nachweis von Sepsis-Erregern wurde daher ein auf der Ganzzellhybridisierung beruhendes Verfahren entwickelt, das eine Identifizierung innerhalb von 2 - 3 Stunden nach Wachstum des Keims in der Blutkultur mit einer Spezifität von 100 % ermöglicht. Der hierfür entwickelte Sondensatz kann zur Identifikation von über 95 % der Sepsis-verursachenden Erregern verwendet werden (37). Zur Überprüfung der dargestellten Sonden wurden 104 septikämische Patienten untersucht, die einen positiven Wachstumsindex in den Blutkulturen aufwiesen.

Aus den Blutkulturen wird sofort nach einem positiven Befund steril eine Probe entnommen. Ein Teil dieser Probe wird jeweils für eine Subkultur auf Agarplatten, für das Anlegen einer Gram-Färbung und für die Ganzzellhybridisierung verwendet. Je nach dem Ergebnis der Gram-Färbung oder nach Bedarf kann eine Auswahl der in Tabelle 4 aufgeführten Sonden zur Identifizierung von Sepsis stattfinden. Es können jedoch auch alle für die Sepsisdiagnose ausgewiesenen Sonden für die nachfolgende Ganzzellhybridisierung verwendet werden.

Zum Nachweis auf Objektträgern werden je nach Anzahl der Sonden mehrere Felder mit je 10 µl aus der positiven Blutkultur betropft und nach Lufttrocknung hitzefixiert. Nach einer aufsteigenden EtOH-Reihe (s. Beispiel 3) werden Gram-positive Bakterien 10 min mit einer Lösung aus 1 mg/ml Lysozym und 5 min mit einer Lösung aus 10 µg/ml Lysostaphin permeabilisiert. Anschließend werden die Proben wie in Beispiel 3 beschrieben hybridisiert und mit zur Ermittlung der Gesamtzahl an Mikroorganismen mit DAPI gefärbt.

Die Untersuchungsergebnisse der Proben von 104 septikämischen Patienten sind in Tabelle 11 zusammengefaßt.

Tabelle 11:

| Gram-Färbung | | Ganzzellhybridisierung | | Kultur | |
|---|---|---|---|---|---|
| Morphologie | n | Sonde | n | | n |
| Gram positive Haufenkokken | 57 | Bacteria | 57 | Wachstum auf Subkulturplatten | 57 |
| | | Staphylococcus | 57 | | |
| | | Staphylococcus aureus | 17 | Staphylococcus aureus | 17 |
| | | | | Koagulase-negative Staphylokokken | 40 |
| Gram positive Kettenkokken | 17 | Bacteria | 17 | Wachstum auf Subkulturplatten | 17 |
| | | Streptococcus | 8 | | |
| | | S. pyogenes | 0 | S. pyogenes | 0 |
| | | S. agalactiae | 0 | S. agalactiae | 0 |
| | | Enterococcus ssp. | 8 | Enterococcus ssp. | 8 |
| | | S. pneumoniae | 4 | S. pneumoniae | 4 |
| | | Keine spezifische Sonde gebunden | 1 | Streptococcus viridans Gruppe | 4 |
| | | | | Lactococcus lactis | 1 |
| Gram positive Stäbchen | 1 | Bacteria | 1 | Wachstum auf Subkulturplatten | 1 |
| | | Keine Spezies-spezifische Sonde gebunden | 1 | Propionibacterium acnes | 1 |
| Gram negative Stäbchen | 27 | Bacteria | 27 | Wachstum auf Subkulturplatte | 27 |

(fortgesetzt)

| Gram-Färbung | | Ganzzellhybridisierung | | Kultur | |
|---|---|---|---|---|---|
| | | Enterobacteriaceae | 22 | | |
| | | K. pneumoniae | 2 | Klebsiella pneumoniae | 2 |
| | | Pseudomonas aeruginosa | 3 | Pseudomonas aeruginosa | 3 |
| | | *Stenotrophomonas maltophilia* | 0 | *Stenotrophomonas maltophilia* | 0 |
| | | Keine Spezies-spezifische Sonde gebunden | 2 | Moraxella osloensis | 1 |
| | | | | Bacteroides fragilis | 1 |
| Hefe | | Fungus | 2 | Hefen auf der Agarplatte | 2 |
| | | Candida albicans | 2 | Candida albicans | 2 |
| | | Candida glabrata | 0 | Candida glabrata | 0 |
| | | Candida parapsilosis | 0 | Candida parapsilosis | 0 |
| | | Candida *krusei* | 0 | Candida *krusei* | 0 |

In 100 von 104 Patientenproben konnten die in den Blutkulturen gewachsenen Bakterien mindestens bis auf Gruppenebene identifiziert werden (96,2 % Sensitivität). Die Identifikation stimmte hierbei zu 100 % mit der klassischen kulturbasierten Identifikation überein (100 % Spezifität) und war in allen Fällen bereits 3 h nach der automatischen Meldung des Blutkultursystems abgeschlossen. Mit Hilfe des ausgewiesenen Verfahrens konnte somit eine Zeitersparnis gegenüber der gängigen Identifikation von 26 bis 46 Stunden erzielt werden. Aufgrund dieser schnellen und verläßlichen Ergebnisse kann eine spezifische Therapie bereits einen bis zwei Tage früher eingeleitet werden als bisher üblich.

Mit dem zur Identifikation von Sepsis ausgewiesenen Sondensatz lassen sich insgesamt eine Reihe wichtiger therapeutischer und diagnostischer Fragestellungen lösen.

Zum einen lassen sich schnell Koagulase negative Staphylococcen, die in über 90 % der Fälle Kontaminantionen darstellen, von klinisch relevanten *Staphylococcus aureus* Stämmen unterscheiden. Insbesondere zur Identifikation von Methicillin-resistenten S. aureus Isolaten, die oft eine fragliche Koagulasereaktion aufweisen, eignet sich die dargestellte Methode hervorragend. Auch die schnelle und sichere Abgrenzung von *Staphylococcus haemolyticus,* der oft eine schwach Koagulasereaktion aufweist, von *Staphylococcus aureus* gelingt mit der Ganzzellhybridisierung problemlos. Bei den Streptococcen ist die Differenzierung von S. *pneumoniae* von den Enterococcen von therapeutischer Bedeutung (Penicillin G versus Ampicillin + Gentamicin). Auch die schnelle Unterscheidung bei den Gram-negativen Stäbchen zieht therapeutische Konsequenzen nach sich. Für *Pseudomonas aeruginosa* ist eine andere Therapieform zu empfehlen als für *Stenotrophomonas maltophilia,* der wiederum mit einer anderen Antibiotikakombination behandelt wird als die Gruppe der Enterobacteriaceae.

Bei den Candida-Infektionen ist vor allem die Unterscheidung *C. krusei* von den anderen Candidaspezies von Bedeutung, da *C. krusei* eine primäre Fluconazolresistenz aufweist (35).

Zusammenfassend kann daher gesagt werden, daß das in der Erfindung dargestellte Verfahren gegenüber anderen kultur- und molekularbiologisch basierten Verfahren zur Diagnose von Sepsiserregern verschiedene Vorteile aufweist, da es schnell, einfach durchzuführen und vergleichsweise billig ist, wodurch es für Routinanwendungen geeignet ist.

Beispiel 4:

Gesamtzellhybridisierung in Mikrotiterplatten mit Enzym-markierten Sonden zum Nachweis von Mikroorganismen

Der neuartige Nachweis von Mikroorganismen in Mikrotiterplatten hat gegenüber der in-situ-Hybridisierung auf Objektträgern den Vorteil, daß die analysierbare Probenmenge erhöht werden kann und damit die Sensitivität des Gesamtsystems steigt. Ferner läßt sich diese Anwendung automatisieren und die Auswertung kann in weit verbreiteten Mikrotiter-Lesegeräten erfolgen.

Zur Etablierung dieser Anwendung wurden Escherichia coli (DH5α) und Pseudomonas aeruginosa (ATCC 27853) Flüssig-Kulturen abzentrifugiert und mit PBS resuspendiert. Zum Fixieren wurden 2 Volumenanteile einer 4 % Paraformaldehyd-Lösung hinzugefügt und bei 4 °C für 3 Stunden inkubiert. Danach wurden die Bakterien erneut abzentrifugiert und mit PBS gewaschen. Dann wurden die Bakterien in einer EtOH-PBS (1 : 1 Vol-%) Lösung bis zum Gebrauch bei -20 °C gelagert. Die Immobilisierung gelang durch Beschickung von Mikrotiterplatten (z.B. beschichtete Maxi-Sorp Platten der Firma Nunc) mit dieser Lösung und Inkubation bei 37 °C bis zur Austrocknung.

Zur Permeabilisierung wurden in jede Vertiefung der Platte 10 mM Natriumcitrat gegeben und es folgte eine 15minütige Behandlung in einer Mikrowelle (700 Watt). Es schloß sich eine kombinierte Behandlung mit Proteinase K und Lysozym (jeweils 0,1 mg/ml) über 15 Minuten bei 37 °C an. Abschließend wurden die Bakterien einer aufsteigenden EtOH-Reihe (80 und 96 % für jeweils 3 Minuten) ausgesetzt.

Zur Hybridisierung wurden die Proben mit einem Hybridisierungspuffer bestehend aus 0,9 M NaCl, 0,02 M Tris/HCl pH 8,0, 0,01 % SDS, 20 % Formamid und 5 ng Sonde/$\mu$l Hybridisierungspuffer 90 Minuten bei 46 °C inkubiert. Als Sonde wurde eine Digoxigenin markierte Enterobacteriaceae Sonde (SEQ ID NO. 3) eingesetzt. Anschließend erfolgte ein 1 5-minütiger stringenter Waschschritt bei 46 °C in einem Puffer, der aus 0,225 M NaCl, 0,02 M Tris/HCl pH 8,0 und 0,01 % SDS bestand. Danach wurde der Puffer abgesaugt und die Vertiefungen mit 10 % fötalem Kälberserum (FCS) in PBS für 10 Minuten bei Raumtemperatur geblockt. Das FCS wurde dann abpipettiert und die Platte bei Raumtemperatur trocknen gelassen.

Zum spezifischen Nachweis von E. coli mit der Enterobacteriaceae-Sonde wurde dann bei Raumtemperatur mit einem Anti-Digoxigenin-Peroxidase Fab-Fragment haltigen Puffer inkubiert. Der Puffer enthielt 150 mM NaCl, 100 mM Tris/HCl pH 7,5, 2 % Blocking Reagenz (Boehringer, in warmen Maleinsäurepuffer, 100 mM Malein, 150 mM NaCl, pH 7,5, gelöst) und 0,15 U Anti-Digoxigenin-POD Fab-Fragment. Nach einer Stunde wurde diese Lösung durch einen Waschpuffer mit 150 mM NaCl und 100 mM Tris/HCl pH 7,5 ersetzt. Nach 1 5-minütiger Inkubation bei Raumtemperatur wurde erneut mit 10 % FCS geblockt. Schließlich wurde o-Phenylendiamindihydrochlorid als Peroxidase-Substrat hinzugefügt und lichtgeschützt bei Raumtemperatur inkubiert. Nach 30 Minuten wurde die Reaktion durch Hinzufügen von 3 M HCl gestoppt und die Farbreaktion im Mikrotiterplatten-Lesegerät bei 490 nm quantifiziert. Der Mittelwert der OD für E. coli betrug 0,5, wohingegen bei P. aeruginosa im Durchschnitt lediglich eine OD von 0,174 gemessen wurde.

Mit Hilfe der durchgeführten Versuche gelang erstmalig der Nachweis von Mikroorganismen in Mikrotiterplatten mit Hilfe einer Gesamtzellhybridisierung. Das dargestellte Verfahren verwendet kostengünstige Standardreagenzien, die sich gut für den täglichen Einsatz im Labor eignen.

**Literaturzitate**

1. Amann, R. I., B. J. Binder, R. J. Olson, S. W. Chisholm, R. Devereux, and D. A. Stahl. 1990. Combination of 16S rRNA-targeted oligonucleotide probes with flow cytometry for analyzing mixed microbial populations. Appl Environ Microbiol. 56:1919-25.

2. Amann, R. I., W. Ludwig, and K. H. Schleifer. 1995. Phylogenetic identification and in situ detection of individual microbial cells without cultivation. Microbiol Rev. 59:143-69.

3. Amann, R. I., B. Zarda, D. A. Stahl, and K. H. Schleifer. 1992. Identification of individual prokaryotic cells by using enzyme-labeled, rRNA-targeted oligonucleotide probes. Appl Environ Microbiol. 58:3007-11.

4. Arnoldi, J., C. Schluter, M. Duchrow, L. Hubner, M. Ernst, A. Teske, H. D. Flad, J. Gerdes, and E. C. Bottger. 1992. Species-specific assessment of Mycobacterium leprae in skin biopsies by in situ hybridization and polymerase chain reaction. Lab Invest. 66:618-23.

5. Brook, I., and E. H. Frazier. 1995. Clinical and microbiological features of necrotizing fasciitis. J Clin Microbiol. 33:2382-7.

6. Daschner, F. D., H. Langmaak, B. Ahlborn, and A. Kümmel. 1983. Kontamination oder Sepsis. Münch Med Wochenschr. 125:849-850.

7. Davis, P. B., M. Drumm, and M. W. Konstan. 1996. Cystic fibrosis. Am J Respir Crit Care Med. 154:1229-56.

8. DeLong, E. F., G. S. Wickham, and N. R. Pace. 1989. Phylogenetic stains: ribosomal RNA-based probes for the identification of single cells [published erratum appears in Science 1989 Sep 22;245(4924):1312]. Science. 243:1360-3.

9. Dickson, E. F., A. Pollak, and E. P. Diamandis. 1995. Time-resolved detection of lanthanide luminescence for ultrasensitive bioanalytical assays. J Photochem Photobiol B. 27:3-19.

10. Edman, J. C., J. A. Kovacs, H. Masur, D. V. Santi, H. J. Elwood, and M. L. Sogin. 1988. Ribosomal RNA sequence shows Pneumocystis carinii to be a member of the fungi. Nature. 334:519-22.

11. Forsgren, J., A. Samuelson, A. Ahlin, J. Jonasson, B. Rynnel-Dagoo, and A. Lindberg. 1994. Haemophilus influenzae resides and multiplies intracellularly in human adenoid tissue as demonstrated by in situ hybridization and bacterial viability assay. Infect Immun. 62:673-9.

12. Fox, G. E., K. J. Pechman, and C. R. Woese. 1977. Comparative cataloging of 16S ribosomal ribonucleic acid: molecular approach to procaryotic systematics. Int. J. System. Bacteriol. 27:44-57.

13. Frischer, M. E., P. J. Floriani, and S. A. Nierzwicki-Bauer. 1996. Differential sensitivity of 16S rRNA targeted oligonucleotide probes used for fluorescence in situ hybridization is a result of ribosomal higher order structure. Can J Microbiol. 42:1061-71.

14. Fuchs, B. M., G. Wallner, W. Beisker, I. Schwippl, W. Ludwig, and R. Amann. 1998. Flow cytometric analysis of the in situ accessibility of Escherichia coli 16S rRNA for fluorescently labeled oligonucleotide probes. Appl Environ Microbiol. 64:4973-82.

**15.** Gall, J. G., and M. L. Pardue. 1969. Formation and detection of RNA-DNA hybrid molecules in cytological preparations. Proc Natl Acad Sci US A. 63:378-83.

16. Giovannoni, S. J., E. F. DeLong, G. J. Olsen, and N. R. Pace. 1988. Phylogenetic group-specific oligodeoxynucleotide probes for identification of single microbial cells [published erratum appears in J Bacteriol 1988 May;170 (5):2418]. J Bacteriol. 170:720-6.

17. Govan, J. R., and V. Deretic. 1996. Microbial pathogenesis in cystic fibrosis: mucoid Pseudomonas aeruginosa and Burkholderia cepacia. Microbiol Rev. 60:539-74.

18. Haase, A. T., L. Stowring, J. D. Harris, B. Traynor, P. Ventura, R. Peluso, and M. Brahic. 1982. Visna DNA synthesis and the tempo of infection in vitro. Virology. 119:399-410.

19. Harrison, P. R., D. Conkie, N. Affara, and J. Paul. 1974. In situ localization of globin messenger RNA formation. I. During mouse fetal liver development. J Cell Biol. 63:402-13.

20. Johansen, H. K., T. A. Kovesi, C. Koch, M. Corey, N. Hoiby, and H. Levison. 1998. Pseudomonas aeruginosa and Burkholderia cepacia infection in cystic fibrosis patients treated in Toronto and Copenhagen. Pediatr Pulmonol. 26:89-96.

21. John, H. A., M. Patrinou-Georgoulas, and K. W. Jones. 1977. Detection of myosin heavy chain mRNA during myogenesis in tissue culture by in vitro and in situ hybridization. Cell. 12:501-8.

22. Kahl, B., M. Herrmann, A. S. Everding, H. G. Koch, K. Becker, E. Harms, R. A. Proctor, and G. Peters. 1998. Persistent infection with small colony variant strains of Staphylococcus aureus in patients with cystic fibrosis. J Infect Dis. 177:1023-9.

23. Kessler, C. 1994. Non-radioactive analysis of biomolecules. J Biotechnol. 35:165-89.

24. Krimmer, V., H. Merkert, C. von Eiff, M. Frosch, J. Eulert, J. F. Lohr, J. Hacker, and W. Ziebuhr. 1999. Detection of Staphylococcus aureus and Staphylococcus epidermidis in clinical samples by 16S rRNA-directed in situ hybridization. J Clin Microbiol. 37:2667-73.

25. Kujath, P., and C. Eckmann. 1998. Die nekrotisierende Fasziitis und schwere Weichteilinfektionen durch Gruppe-A-Streptokokken: Diagnose, Therapie und Prognose. Dt Ärztebl. 95:A-408-413.

26. Lambert, H. P., and F. W. O' Grady. 1992. Antibiotic and chemotherapy, 6th ed. Churchill Livingstone, Edinburgh.

27. Lane, D. J., B. Pace, G. J. Olsen, D. A. Stahl, M. L. Sogin, and N. R. Pace. 1985. Rapid determination of 16S ribosomal RNA sequences for phylogenetic analyses. Proc Natl Acad Sci U S A. 82:6955-9.

28. Lebaron, P., P. Catala, C. Fajon, F. Joux, J. Baudart, and L. Bernard. 1997. A new sensitive, whole-cell hybridization technique for detection of bacteria involving a biotinylated oligonucleotide probe targeting rRNA and Tyramide signal amplification. Appl Environ Microbiol. 63:3274-3278.

29. Lee, S., C. Malone, and P. F. Kemp. 1993. Use of multiple 16S rRNA-targeted fluorescent probes to increase signal strengt and measure cellular RNA from natural planktonic bacteria. Mar Ecol Prog Ser. 101:193-201.

30. Lim, E. L., L. A. Amaral, D. A. Caron, and E. F. DeLong. 1993. Application of rRNA-based probes for observing marine nanoplanktonic protists. Appl Environ Microbiol. 59:1647-55.

31. LiPuma, J. J. 1998. Burkholderia cepacia. Management issues and new insights. Clin Chest Med. 19:473-86, vi.

32. Manz, W., R. Amann, W. Ludwig, M. Vancanneyt, and K. H. Schleifer. 1996. Application of a suite of 16S rRNA-specific oligonucleotide probes designed to investigate bacteria of the phylum cytophaga-flavobacter- bacteroides in the natural environment. Microbiology. 142:1097-106.

33. Matsuhisa, A., Y. Saito, H. Ueyama, Y. Aikawa, and T. Ohono. 1994. Detection of Staphylococci in mouse phagocytic cells by in situ hybridization using biotinylated DNA probes. Biotech Histochem. 69:31-7.

34. Meier, H., Koob, C., Ludwig, W, et al. 1997. Detection of enterococci with rRNA targeted DNA probes and their use for hygienic drinking water control. Water Sci. Technol. 35:437-444.

35. Rex, J. H., M. A. Pfaller, J. N. Galgiani, M. S. Bartlett, A. Espinel-Ingroff, M. A. Ghannoum, M. Lancaster, F. C. Odds, M. G. Rinaldi, T. J. Walsh, and A. L. Barry. 1997. Development of interpretive breakpoints for antifungal susceptibility testing: conceptual framework and analysis of in vitro-in vivo correlation data for fluconazole, itraconazole, and candida infections. Subcommittee on Antifungal Susceptibility Testing of the National Committee for Clinical Laboratory Standards [see comments]. Clin Infect Dis. 24:235-47.

36. Roller, C., M. Wagner, R. Amann, W. Ludwig, and K. H. Schleifer. 1994. In situ probing of gram-positive bacteria with high DNA G + C content using 23S rRNA-targeted oligonucleotides [published erratum appears in Microbiology 1995 May;141(Pt 5):1267]. Microbiology. 140:2849-58.

37. Rosenthal, E. J. K. 1986. Septikämie-Erreger 1983-1985. Ergebnisse einer multizentrischen Studie. Dtsch Med Wochenschr. 49:1-8.

**38.** Sandhu, G. S., B. C. Kline, L. Stockman, and G. D. Roberts. 1995. Molecular probes for diagnosis of fungal infections [published erratum appears in J Clin Microbiol 1996 May;34(5):1350]. J Clin Microbiol. 33:2913-9.

39. Schleifer, K. H., and O. Kandler. 1972. Peptidoglycan types of bacterial cell walls and their taxonomic implications. Bacteriol Rev. 36:407-77.

40. Schonhuber, W., B. Fuchs, S. Juretschko, and R. Amann. 1997. Improved sensitivity of whole-cell hybridization by the combination of horseradish peroxidase-labeled oligonucleotides and tyramide signal amplification. Appl Environ Microbiol. 63:3268-73.

41. Sogin, S. J., M. L. Sogin, and C. R. Woese. 1971. Phylogenetic measurement in procaryotes by primary structural characterization. J Mol Evol. 1:173-84.

42. Stahl, D. A., and R. Amann. 1991. Development and application of nucleic acid probes, p. 205-248. In E. Stackebrand, and M. Goodfellow (ed.), Nucleic Acid Techniques in bacterial systematics. John Wiley & sons, Chichester, England.

**43.** Stamenkovic, I., and P. D. Lew. 1984. Early recognition of potentially fatal necrotizing fasciitis. The use of frozen-section biopsy. N Engl J Med. 310:1689-93.

**44.** Trebesius, K., R. Amann, W. Ludwig, K. Mühlegger, and K.-H. Schleifer. 1994. Identification of whole fixed bacterial cells with nonradioactive 23S rRNA-targeted polynucleotide probes. Appl Environ Microbiol. 60:3228-3235.

45. Trebesius, K., D. Harmsen, A. Rakin, J. Schmelz, and J. Heesemann. 1998. Development of rRNA-targeted PCR and in situ hybridization with fluorescently labelled oligonucleotides for detection of Yersinia species. J Clin Microbiol. 36:2557-64.

**46.** Tümmler, B., and C. Kiewitz. 1999. Cystic fibrosis: an inherited suseptibility to bacterial respiratory infections. Mol. Med. Today. 5:351-358.

47. Van de Peer, Y., E. Robbrecht, S. de Hoog, A. Caers, P. De Rijk, and R. De Wachter. 1999. Database on the structure of small subunit ribosomal RNA. Nucleic Acids Res. 27:179-83.

48. Wallner, G., R. Amann, and W. Beisker. 1993. Optimizing fluorescent in situ hybridization with rRNA-targeted oligonucleotide probes for flow cytometric identification of microorganisms. Cytometry. 14:136-43.

49. Wetmur, J. G. 1991. DNA probes: applications of the principles of nucleic acid hybridization. Crit Rev Biochem Mol Biol. 26:227-59.

50. Zach, M. S. 1990. Lung disease in cystic fibrosis--an updated concept. Pediatr Pulmonol. 8:188-202.

51. Zarda, B., R. Amann, G. Wallner, and K. H. Schleifer. 1991. Identification of single bacterial cells using digoxigenin-labelled, rRNA-targeted oligonucleotides. J Gen Microbiol. 137:2823-30.

**Patentansprüche**

1. Verfahren zur direkten Identifizierung von Mikroorganismen in einer biologischen Probe für die Diagnose von Sepsis, umfassend die Schritte:

    (a) Aufteilen einer zu untersuchenden biologischen Probe in mehrere Teilproben,
    (b) Vorbereiten der Teilproben für das Nachweisverfahren, wobei in der Probe vorhandene Mikroorganismen immobilisiert werden,
    (c) Inkontaktbringen von mehreren der vorbereiteten Teilproben mit jeweils einer oder mehreren markierten Hybridisierungssonden, die einen Hybridisierungsbereich mit einer Länge bis 100 nt aufweisen, wobei

(i) jeweils unterschiedliche Hybridisierungssonden oder Kombinationen von Hybridisierungssonden für einzelne Teilproben verwendet werden,
(ii) die Hybridisierungssonden unter gleichen Hybridisierungsbedingungen angewendet werden,
(iii) die Hybridisierungssonden einen Hybridisierungsbereich enthalten, der zu Zielsequenzen komplementär ist, ermittelt aus Nukleinsäuren, die spezifisch für mögliche in der Probe vorhandene Mikroorganismen sind, wobei die Mikroorganismen für Sepsis verantwortlich sind, und
(iv) die Zielsequenzen so ermittelt werden, dass sie im Inneren der Zelle für eine Hybridisierung verfügbar sind, und

(d) Nachweisen von gebundenen Markierungen in den Teilproben,
wobei ein Satz von markierten Hybridisierungssonden verwendet wird, umfassend:

(I) gruppenspezifische Sonden, jede Sonde enthaltend eine des Sequenzen gemäß SEQ ID NO. 2, 3, 5, 6, 17 und 38 oder eine dazu mindestens 85 % identische Sequenz, und
(II) speziesspezifische Sonden, jede sonde enthaltend eine des Sequenzen gemäß SEQ ID NO. 11, 12, 13, 14, 15, 16, 18, 19, 20, 21, 22, 23, 24, 25, 36 und 37 oder eine dazu mindestens 85 % identische Sequenz.

2. Verfahren nach Anspruch 1,
**dadurch gekennzeichnet,**
**dass** die biologische Probe aus klinischen Proben ausgewählt wird.

3. Verfahren nach Anspruch 1 oder 2,
**dadurch gekennzeichnet,**
**dass** Schritt (a) eine Aufbewahrung der Probe in einem Medium umfasst.

4. Verfahren nach einem der vorhergehenden Ansprüche,
**dadurch gekennzeichnet,**
**dass** Schritt (a) eine Voruntersuchung der Probe oder/und von Teilproben umfasst.

5. Verfahren nach einem der vorhergehenden Ansprüche,
**dadurch gekennzeichnet,**
**dass** Schritt (a) eine mikroskopische Untersuchung oder/und eine Gram-Färbung der Probe oder/und von Teilproben umfasst.

6. Verfahren nach einem der vorhergehenden Ansprüche,
**dadurch gekennzeichnet,**
**dass** die einzelnen Teilproben auf jeweils separate Träger oder in jeweils separate Gefäße überführt werden.

7. Verfahren nach Anspruch 6,
**dadurch gekennzeichnet,**
**dass** die einzelnen Teilproben in jeweils separate optisch transparente Reaktionsgefäße überführt werden.

8. Verfahren nach einem der vorhergehenden Ansprüche,
**dadurch gekennzeichnet,**
**dass** Schritt (b) ein Immobilisieren der Mikroorganismen durch Trocknung, Filtration oder/und Zentrifugation umfasst.

9. Verfahren nach einem der vorhergehenden Ansprüche,
**dadurch gekennzeichnet,**
**dass** Schritt (b) weiterhin ein Fixieren der Mikroorganismen umfasst.

10. Verfahren nach einem der vorhergehenden Ansprüche,
**dadurch gekennzeichnet,**
**dass** Schritt (b) weiterhin eine Permeabilisierung der Mikroorganismen umfasst.

11. Verfahren nach Anspruch 10,
**dadurch gekennzeichnet,**
**dass** die Permeabilisierung durch enzymatische, chemische, mechanische oder/und thermische Behandlung der Mikroorganismen erfolgt.

**12.** Verfahren nach Anspruch 11,
**dadurch gekennzeichnet,**
**dass** die Permeabilisierung von Gram-positiven Mikroorganismen durch Behandlung mit Lysozym und Lysostaphin erfolgt.

**13.** Verfahren nach einem der vorhergehenden Ansprüche,
**dadurch gekennzeichnet,**
**dass** als Hybridierungssonden in Schritt (c) Oligonukleotide verwendet werden, die einen Hybridisierungsbereich mit einer Länge von 17 bis 22 nt aufweisen.

**14.** Verfahren nach einem der vorhergehenden Ansprüche,
**dadurch gekennzeichnet,**
**dass** in Schritt (c) direkt oder/und indirekt markierte Hybridisierungssonden verwendet werden.

**15.** Verfahren nach Anspruch 14,
**dadurch gekennzeichnet,**
**dass** in Schritt (c) fluoreszenz- oder/und enzymmarkierte Hybridisierungssonden verwendet werden.

**16.** Verfahren nach einem der vorhergehenden Ansprüche,
**dadurch gekennzeichnet,**
**dass** in Schritt (c) Hybridisierungssonden verwendet werden, die gegen zugängliche Bereiche ribosomaler RNA intakter Ribosomen innerhalb der nachzuweisenden Mikroorganismen gerichtet sind.

**17.** Verfahren nach einem der vorhergehenden Ansprüche,
**dadurch gekennzeichnet,**
**dass** die Hybridisierung bei für alle Sonden gleichen Bedingungen hinsichtlich Temperatur und Zeit durchgeführt wird.

**18.** Verfahren nach einem der vorhergehenden Ansprüche,
**dadurch gekennzeichnet,**
**dass** nach der Hybridisierung ein Waschen bei einer gegenüber den Hybridisierungsbedingungen verringerter Salzkonzentration erfolgt.

**19.** Verfahren nach einem der vorhergehenden Ansprüche,
**dadurch gekennzeichnet,**
**dass** weiterhin eine Antibiotikaresistenz-Bestimmung durch eine in situ Hybridisierung durchgeführt wird.

**20.** Reagenzienkit zur Identifizierung von Mikroorganismen in biologischen Proben für die Diagnose von Sepsis umfassend einen Satz von markierten Hybridisierungssonden, die einen Hybridisierungsbereich mit einer Länge bis 100 nt aufweisen, wobei die Hybridisierungssonden unter gleichen Hybridisierungsbedingungen angewendet werden, und jeweils unterschiedliche Hybridisierungssonden einen Hybridisierungsbereich enthalten, der zu Zielsequenzen komplementär ist, ermittelt aus Nukleinsäuren, die spezifisch für mögliche in der Probe vorhandene Mikroorganismen verantwortlich für Sepsis, sind und wobei die Sonden so ausgewählt sind, dass sie im Inneren der Zelle für eine Hybridisierung verfügbar sind, wobei der Satz von markierten Hybridisierungssonden umfasst:

(I) gruppenspezifische Sonden, jede Sonde enthaltend eine der Sequenzen gemäß SEQ ID NO. 2, 3, 5, 6, 17 und 38 oder eine dazu mindestens 85 % identische Sequenz, und
(II) speziesspezifische Sonden, jede Sonde enthaltend eine des Sequenzen gemäß SEQ ID NO. 11, 12, 13, 14, 15, 16, 18, 19, 20, 21, 22, 23, 24, 25, 36 und 37 oder eine dazu mindestens 85 % identischen Sequenz.

**21.** Verwendung des Reagenzienkits nach Anspruch 20 in einem Verfahren nach einem der Ansprüche 1 bis 19.

**22.** Verwendung eines Satzes von Oligonukleotiden, die einen Hybridisierungsbereich zur Hybridisierung von mikrobiellen Zielsequenzen enthalten, wobei der Hybridisierungsbereich eine Länge bis 100 nt aufweist und wobei der Hybridisierungsbereich jedes Oligonucleotids eine des Sequenzen gemäß SEQ ID NO. 2, 3, 5, 6, 11, 12, 13, 14, 15, 16, 17, 18, 19, 20, 21, 22, 23, 24, 25, 36, 37 und 38 oder eine dazu mindestens 85 % identische Sequenz enthält, in einem Verfahren nach einem der Ansprüche 1 bis 19.

**23.** Verwendung nach Anspruch 22,
**dadurch gekennzeichnet,**
**dass** das Oligonukleotid eine Markierungsgruppe trägt.

**Claims**

**1.** Method for the direct identification of microorganisms in a biological sample for the diagnosis of sepsis, comprising the steps:

a) dividing a biological sample to be investigated into a plurality of part-samples,
b) preparing the part-samples for the detection method, with immobilization of microorganisms present in the sample,
c) contracting a plurality of the prepared part-samples with in each case one or more labelled hybridization probes which have a hybridization region with a length of up to 100 nt, where

i) in each case different hybridization probes or combinations of hybridization probes are used for individual part-samples,
ii) the hybridization probes are employed under identical hybridization conditions,
iii) the hybridization probes comprise a hybridization region which is complementary to target sequences ascertained from nucleic acids which are specific for possible microorganisms present in the sample, the microorganisms being responsible for sepsis, and
iv) the target sequences are ascertained in such a way that they are available in the interior of the cell for a hybridization, and

d) detecting bound labels in the part-samples, where a set of labelled hybridization probes is used, comprising:

i) group-specific probes, each probe comprising one of the sequences as shown in SEQ ID NO. 2, 3, 5, 6, 17 and 38 or a sequence which is at least 85% identical thereto and
ii) species-specific probes, each probe comprising one of the sequences as shown in SEQ ID NO. 11, 12, 13, 14, 15, 16, 18, 19, 20, 21, 22, 23, 24, 25, 36 and 37 or a sequence which is at least 85% identical thereto.

**2.** Method according to Claim 1, **characterized in that** the biological sample is selected from clinical samples.

**3.** Method according to Claim 1 or 2, **characterized in that** step (a) comprises a storage of the sample in a medium.

**4.** Method according to one of the preceding claims, **characterized in that** step (a) comprises a preliminary investigation of the sample or/and of part-samples.

**5.** Method according to one of the preceding claims, **characterized in that** step (a) comprises a microscopic examination or/and a Gram stain of the sample or/and of part-samples.

**6.** Method according to one of the preceding claims, **characterized in that** the individual part-samples are transferred to separate carriers in each case or into separate vessels in each case.

**7.** Method according to Claim 6, **characterized in that** the individual part-samples are transferred into separate optically transparent reaction vessels in each case.

**8.** Method according to one of the preceding claims, **characterized in that** step (b) comprises an immobilization of the microorganisms by drying, filtration or/and centrifugation.

**9.** Method according to one of the preceding claims, **characterized in that** step (b) additionally comprises a fixation of the microorganisms.

**10.** Method according to one of the preceding claims, **characterized in that** step (b) additionally comprises a permeabilization of the microorganisms.

**11.** Method according to Claim 10, **characterized in that** the permeabilization is effected by enzymatic, chemical,

EP 1 232 286 B1

mechanical and/or thermal treatment of the microorganisms.

12. Method according to Claim 11, **characterized in that** Gram-positive microorganisms are permeabilized by treatment with lysozyme and lysostaphin.

13. Method according to one of the preceding claims, **characterized in that** the hybridization probes used in step (c) are oligonucleotides which have a hybridization region with a length of from 17 to 22 nt.

14. Method according to any of the preceding claims, **characterized in that** directly or/and indirectly labelled hybridization probes are used in step (c).

15. Method according to Claim 14, **characterized in that** fluorescent- or/and enzyme-labelled hybridization probes are used in step (c).

16. Method according to one of the preceding claims, **characterized in that** the hybridization probes used in step (c) are directed against accessible regions of ribosomal RNA of intact ribosomes within the microorganisms to be detected.

17. Method according to one of the preceding claims, **characterized in that** the hybridization is carried out with the same conditions in relation to temperature and time for all probes.

18. Method according to one of the preceding claims, **characterized in that** the hybridization is followed by a washing with a salt concentration which is reduced compared wit the hybridization conditions.

19. Method according to one of the preceding claims, **characterized in that** an antibiotic resistance determination by an in situ hybridization is additionally carried out.

20. Reagent kit for identifying microorganisms in biological samples for the diagnosis of sepsis comprising a set of labelled hybridization probes which have a hybridization region with a length of up to 100 nt, where the hybridization probes are employed under identical hybridization conditions, and different hybridization probes in each case comprise a hybridization region which is complementary to target sequences ascertained from nucleic acids which are specific for possible microorganisms responsible for sepsis present in the sample, and where the probes are selected such that they are available for a hybridization in the interior of the cell, where the set of labelled hybridization probes comprises:

i) group-specific probes, each probe comprising one of the sequences as shown in SEQ ID NO. 2, 3, 5, 6, 17 and 38 or a sequence which is at least 85% identical thereto and
ii) species-specific probes, each probe comprising one of the sequences as shown in SEQ ID NO. 11, 12, 13, 14, 15, 16, 18, 19, 20, 21, 22, 23, 24, 25, 36 and 37 or a sequence which is at least 85% identical thereto.

21. Use of the reagent kit according to Claim 20 in a method according to one of Claims 1 to 19.

22. Use of a set of oligonucleotides which comprise a hybridization region for the hybridization of microbial target sequences, where the hybridization region has a length of up to 100 nt and where the hybridization region of each oligonucleotide comprises one of the sequences as shown in SEQ ID NO. 2, 3, 5, 6, 11, 12, 13, 14, 15, 16, 17, 18, 19, 20, 21, 22, 23, 24, 25, 36, 37 and 38 or a sequence which is at least 85% identical thereto in a method according to one of Claims 1 to 19.

23. Use according to Claim 22, **characterized in that** the oligonucleotide carries a labelling group.

## Revendications

1. Procédé d'identification de micro-organismes dans un échantillon biologique en vue du diagnostic d'une sepsie, lequel procédé comprend les étapes qui consistent à :

a) répartir un échantillon biologique à étudier entre plusieurs échantillons partiels,
b) préparer les échantillons partiels en vue de l'opération de détection en immobilisant les micro-organismes présents dans l'échantillon,

c) mettre plusieurs des échantillons partiels préparés en contact avec une ou plusieurs sondes d'hybridation marquées dont la plage d'hybridation a une longueur qui peut atteindre 100 nt,

i) différentes sondes d'hybridation ou combinaisons de sondes d'hybridation étant utilisées pour les différents échantillons partiels,

ii) les sondes d'hybridation étant utilisées dans les mêmes conditions d'hybridation,

iii) les sondes d'hybridation contenant une plage d'hybridation complémentaire de séquences visées déterminées à partir d'acides nucléiques qui sont spécifiques à des micro-organismes qui peuvent être présents dans l'échantillon, les micro-organismes étant responsables de la sepsie, et

iv) les séquences visées étant déterminées de manière à être accessibles à une hybridation à l'intérieur de la cellule,

d) détecter des marqueurs liés aux échantillons partiels en utilisant un ensemble de sondes d'hybridation marquées qui comprennent :

i) des sondes spécifiques à un groupe, chaque sonde contenant une des séquences SEQ ID No : 2, 3, 5, 6, 17 et 38 ou une séquence qui leur est identique à au moins 85 % et

ii) des sondes spécifiques à une substance, chaque sonde contenant une des séquences SEQ ID No : 11, 12, 13, 14, 15, 16, 18, 19, 20, 21, 22, 23, 24, 25, 36 et 37 ou une séquence qui leur est identique à au moins 85 %.

2. Procédé selon la revendication 1, **caractérisé en ce que** l'échantillon biologique est sélectionné parmi des échantillons cliniques.

3. Procédé selon les revendications 1 ou 2, **caractérisé en ce que** l'étape (a) comprend la conservation de l'échantillon dans un fluide.

4. Procédé selon l'une des revendications précédentes, **caractérisé en ce que** l'étape (a) comprend une préétude de l'échantillon et/ou des échantillons partiels.

5. Procédé selon l'une des revendications précédentes, **caractérisé en ce que** l'étape (a) comprend un examen microscopique et/ou une coloration gram de l'échantillon et/ou des échantillons partiels.

6. Procédé selon l'une des revendications précédentes, **caractérisé en ce que** les différents échantillons partiels sont transférés sur des supports séparés ou dans des récipients séparés.

7. Procédé selon la revendication 6, **caractérisé en ce que** les différents échantillons partiels sont transférés dans des récipients de réaction optiquement transparents et séparés.

8. Procédé selon l'une des revendications précédentes, **caractérisé en ce que** l'étape (b) comprend l'immobilisation des micro-organismes par séchage, filtration et/ou centrifugation.

9. Procédé selon l'une des revendications précédentes, **caractérisé en ce que** l'étape (b) comprend en outre la fixation des micro-organismes.

10. Procédé selon l'une des revendications précédentes, **caractérisé en ce que** l'étape (b) comprend en outre une perméabilisation des micro-organismes.

11. Procédé selon la revendication 10, **caractérisé en ce que** la perméabilisation s'effectue par traitement enzymatique, chimique, mécanique et/ou thermique des micro-organismes.

12. Procédé selon la revendication 11, **caractérisé en ce que** la perméabilisation des micro-organismes gram-positifs s'effectue par traitement au lysozyme et à la lysostaphine.

13. Procédé selon l'une des revendications précédentes, **caractérisé en ce que** comme sonde d'hybridation, on utilise dans l'étape (c) des oligonucléotides dont la plage d'hybridation a une longueur de 17 à 22 nt.

**14.** Procédé selon l'une des revendications précédentes, **caractérisé en ce que** dans l'étape (c), on utilise des sondes d'hybridation marquées directement et/ou indirectement.

**15.** Procédé selon la revendication 14, **caractérisé en ce que** dans l'étape (c), on utilise des sondes d'hybridation marquées par fluorescence et/ou par enzymes.

**16.** Procédé selon l'une des revendications précédentes, **caractérisé en ce que** dans l'étape (c), on utilise des sondes d'hybridation qui sont dirigées contre des zones accessibles d'ARN ribosomal de ribosomes intacts présents à l'intérieur des micro-organismes à détecter.

**17.** Procédé selon l'une des revendications précédentes, **caractérisé en ce que** l'hybridation est réalisée dans les mêmes conditions de température et de durée pour toutes les sondes.

**18.** Procédé selon l'une des revendications précédentes, **caractérisé en ce qu'**après l'hybridation, on réalise un lavage à une concentration de sel plus basse que dans les conditions d'hybridation.

**19.** Procédé selon l'une des revendications précédentes, **caractérisé en ce que** l'on réalise en outre une détermination de la résistance aux antibiotiques par une hybridation in situ.

**20.** Trousse de réactifs destinés à l'identification de micro-organismes dans des échantillons biologiques en vue du diagnostic d'une sepsie, qui comprend un ensemble de sondes d'hybridation marquées dont la plage d'hybridation a une longueur qui peut atteindre 100 nt, les sondes d'hybridation étant utilisées dans les mêmes conditions d'hybridation et des sondes d'hybridation différentes contenant toutes une plage d'hybridation complémentaire de séquences visées déterminées à partir d'acides nucléiques spécifiques à des micro-organismes qui peuvent être présents dans l'échantillon et qui sont responsables de la sepsie, les sondes étant sélectionnées de manière à être accessibles à une hybridation à l'intérieur de la cellule, l'ensemble de sondes d'hybridation marquées comprenant :

  i) des sondes spécifiques à un groupe, chaque sonde contenant une des séquences SEQ ID No : 2, 3, 5, 6, 17 et 38 ou une séquence qui leur est identique à au moins 85 % et
  ii) des sondes spécifiques à une substance, chaque sonde contenant une des séquences SEQ ID No : 11, 12, 13, 14, 15, 16, 18, 19, 20, 21, 22, 23, 24, 25, 36 et 37 ou une séquence qui leur est identique à au moins 85 %.

**21.** Utilisation de la trousse de réactifs selon la revendication 20 dans un procédé selon l'une des revendications 1 à 19.

**22.** Utilisation d'un ensemble d'oligonucléotides qui contiennent une plage d'hybridation qui permet l'hybridation sur des séquences microbiennes visées, la plage d'hybridation ayant une longueur de jusque 100 nt et la plage d'hybridation de chaque oligonucléotide contenant une des séquences SEQ ID No : 2, 3, 5, 6, 11, 12, 13, 14, 15, 16, 17, 18, 19, 20, 21, 22, 23, 24, 25, 36, 37 et 38 ou une séquence qui leur est identique à au moins 85 %, dans un procédé selon l'une des revendications 1 à 19.

**23.** Utilisation selon la revendication 22, **caractérisée en ce que** l'oligonucléotide porte un groupe de marquage.

# IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

## In der Beschreibung aufgeführte Patentdokumente

- US 4486539 A
- EP 0370694 A
- WO 9850583 A
- EP 0497464 A
- WO 9954502 A
- US 456729 A
- WO 8803957 A
- WO 9015157 A
- WO 9100926 A
- US 5679520 A
- EP 9903527 W
- US 4150295 A
- US 4058732 A
- WO 9108490 A
- US 5663049 A
- DE 19955303

## In der Beschreibung aufgeführte Nicht-Patentliteratur

- **SHIMADA et al.** *J. Infect. Chemother,* 1999, vol. 5, 21-31
- **AMANN, R. I. ; B. J. BINDER ; R. J. OLSON ; S. W. CHISHOLM ; R. DEVEREUX ; D. A. STAHL.** Combination of 16S rRNA-targeted oligonucleotide probes with flow cytometry for analyzing mixed microbial populations. *Appl Environ Microbiol.,* 1990, vol. 56, 1919-25
- **AMANN, R. I. ; W. LUDWIG ; K. H. SCHLEIFER.** Phylogenetic identification and in situ detection of individual microbial cells without cultivation. *Microbiol Rev.,* 1995, vol. 59, 143-69
- **AMANN, R. I. ; B. ZARDA ; D. A. STAHL ; K. H. SCHLEIFER.** Identification of individual prokaryotic cells by using enzyme-labeled, rRNA-targeted oligonucleotide probes. *Appl Environ Microbiol.,* 1992, vol. 58, 3007-11
- **ARNOLDI, J. ; C. SCHLUTER ; M. DUCHROW ; L. HUBNER ; M. ERNST ; A. TESKE ; H. D. FLAD ; J. GERDES ; E. C. BOTTGER.** Species-specific assessment of Mycobacterium leprae in skin biopsies by in situ hybridization and polymerase chain reaction. *Lab Invest.,* 1992, vol. 66, 618-23
- **BROOK, I. ; E. H. FRAZIER.** Clinical and microbiological features of necrotizing fasciitis. *J Clin Microbiol.,* 1995, vol. 33, 2382-7
- **DASCHNER, F. D. ; H. LANGMAAK ; B. AHLBORN ; A. KÜMMEL.** Kontamination oder Sepsis. *Münch Med Wochenschr.,* 1983, vol. 125, 849-850
- **DAVIS, P. B. ; M. DRUMM ; M. W. KONSTAN.** Cystic fibrosis. *Am J Respir Crit Care Med.,* 1996, vol. 154, 1229-56
- **DELONG, E. F. ; G. S. WICKHAM ; N. R. PACE.** Phylogenetic stains: ribosomal RNA-based probes for the identification of single cells. *Science,* 22. September 1989, vol. 245 (4924), 1312
- *Science,* vol. 243, 1360-3
- **DICKSON, E. F. ; A. POLLAK ; E. P. DIAMANDIS.** Time-resolved detection of lanthanide luminescence for ultrasensitive bioanalytical assays. *J Photochem Photobiol B.,* 1995, vol. 27, 3-19
- **EDMAN, J. C. ; J. A. KOVACS ; H. MASUR ; D. V. SANTI ; H. J. ELWOOD ; M. L. SOGIN.** Ribosomal RNA sequence shows Pneumocystis carinii to be a member of the fungi. *Nature,* 1988, vol. 334, 519-22
- **FORSGREN, J. ; A. SAMUELSON ; A. AHLIN ; J. JONASSON ; B. RYNNEL-DAGOO ; A. LINDBERG.** Haemophilus influenzae resides and multiplies intracellularly in human adenoid tissue as demonstrated by in situ hybridization and bacterial viability assay. *Infect Immun.,* 1994, vol. 62, 673-9
- **FOX, G. E. ; K. J. PECHMAN ; C. R. WOESE.** Comparative cataloging of 16S ribosomal ribonucleic acid: molecular approach to procaryotic systematics. *Int. J. System. Bacteriol.,* 1977, vol. 27, 44-57
- **FRISCHER, M. E. ; P. J. FLORIANI ; S. A. NIERZWICKI-BAUER.** Differential sensitivity of 16S rRNA targeted oligonucleotide probes used for fluorescence in situ hybridization is a result of ribosomal higher order structure. *Can J Microbiol.,* 1996, vol. 42, 1061-71
- **FUCHS, B. M. ; G. WALLNER ; W. BEISKER ; I. SCHWIPPL ; W. LUDWIG ; R. AMANN.** Flow cytometric analysis of the in situ accessibility of Escherichia coli 16S rRNA for fluorescently labeled oligonucleotide probes. *Appl Environ Microbiol.,* 1998, vol. 64, 4973-82
- **GALL, J. G. ; M. L. PARDUE.** Formation and detection of RNA-DNA hybrid molecules in cytological preparations. *Proc Natl Acad Sci US A.,* 1969, vol. 63, 378-83

- **GIOVANNONI, S. J. ; E. F. DELONG ; G. J. OLSEN ; N. R. PACE.** Phylogenetic group-specific oligodeoxynucleotide probes for identification of single microbial cells. *J Bacteriol,* Mai 1988, vol. 170 (5), 2418
- *J Bacteriol.,* vol. 170, 720-6
- **GOVAN, J. R. ; V. DERETIC.** Microbial pathogenesis in cystic fibrosis: mucoid Pseudomonas aeruginosa and Burkholderia cepacia. *Microbiol Rev.,* 1996, vol. 60, 539-74
- **HAASE, A. T. ; L. STOWRING ; J. D. HARRIS ; B. TRAYNOR ; P. VENTURA ; R. PELUSO ; M. BRAHIC.** Visna DNA synthesis and the tempo of infection in vitro. *Virology,* 1982, vol. 119, 399-410
- **HARRISON, P. R. ; D. CONKIE ; N. AFFARA ; J. PAUL.** In situ localization of globin messenger RNA formation. I. During mouse fetal liver development. *J Cell Biol.,* 1974, vol. 63, 402-13
- **JOHANSEN, H. K. ; T. A. KOVESI ; C. KOCH ; M. COREY ; N. HOIBY ; H. LEVISON.** Pseudomonas aeruginosa and Burkholderia cepacia infection in cystic fibrosis patients treated in Toronto and Copenhagen. *Pediatr Pulmonol.,* 1998, vol. 26, 89-96
- **JOHN, H. A. ; M. PATRINOU-GEORGOULAS ; K. W. JONES.** Detection of myosin heavy chain mRNA during myogenesis in tissue culture by in vitro and in situ hybridization. *Cell,* 1977, vol. 12, 501-8
- **KAHL, B. ; M. HERRMANN ; A. S. EVERDING ; H. G. KOCH ; K. BECKER ; E. HARMS ; R. A. PROCTOR ; G. PETERS.** Persistent infection with small colony variant strains of Staphylococcus aureus in patients with cystic fibrosis. *J Infect Dis.,* 1998, vol. 177, 1023-9
- **KESSLER, C.** Non-radioactive analysis of biomolecules. *J Biotechnol.,* 1994, vol. 35, 165-89
- **KRIMMER, V. ; H. MERKERT ; C. VON EIFF ; M. FROSCH ; J. EULERT ; J. F. LOHR ; J. HACKER ; W. ZIEBUHR.** Detection of Staphylococcus aureus and Staphylococcus epidermidis in clinical samples by 16S rRNA-directed in situ hybridization. *J Clin Microbiol.,* 1999, vol. 37, 2667-73
- **KUJATH, P. ; C. ECKMANN.** Die nekrotisierende Fasziitis und schwere Weichteilinfektionen durch Gruppe-A-Streptokokken: Diagnose, Therapie und Prognose. *Dt Ärztebl.,* 1998, vol. 95, A-408-413
- **LAMBERT, H. P. ; F. W. O' GRADY.** Antibiotic and chemotherapy. 1992
- **LANE, D. J. ; B. PACE ; G. J. OLSEN ; D. A. STAHL ; M. L. SOGIN ; N. R. PACE.** Rapid determination of 16S ribosomal RNA sequences for phylogenetic analyses. *Proc Natl Acad Sci U S A.,* 1985, vol. 82, 6955-9
- **LEBARON, P. ; P. CATALA ; C. FAJON ; F. JOUX ; J. BAUDART ; L. BERNARD.** A new sensitive, whole-cell hybridization technique for detection of bacteria involving a biotinylated oligonucleotide probe targeting rRNA and Tyramide signal amplification. *Appl Environ Microbiol.,* 1997, vol. 63, 3274-3278
- **LEE, S. ; C. MALONE ; P. F. KEMP.** Use of multiple 16S rRNA-targeted fluorescent probes to increase signal strengt and measure cellular RNA from natural planktonic bacteria. *Mar Ecol Prog Ser.,* 1993, vol. 101, 193-201
- **LIM, E. L. ; L. A. AMARAL ; D. A. CARON ; E. F. DELONG.** Application of rRNA-based probes for observing marine nanoplanktonic protists. *Appl Environ Microbiol.,* 1993, vol. 59, 1647-55
- **LIPUMA, J. J.** Burkholderia cepacia. Management issues and new insights. *Clin Chest Med.,* 1998, vol. 19, 473-86
- **MANZ, W. ; R. AMANN ; W. LUDWIG ; M. VANCANNEYT ; K. H. SCHLEIFER.** Application of a suite of 16S rRNA-specific oligonucleotide probes designed to investigate bacteria of the phylum cytophaga-flavobacter- bacteroides in the natural environment. *Microbiology,* 1996, vol. 142, 1097-106
- **MATSUHISA, A. ; Y. SAITO ; H. UEYAMA ; Y. AIKAWA ; T. OHONO.** Detection of Staphylococci in mouse phagocytic cells by in situ hybridization using biotinylated DNA probes. *Biotech Histochem.,* 1994, vol. 69, 31-7
- **MEIER, H. ; KOOB, C. ; LUDWIG, W et al.** Detection of enterococci with rRNA targeted DNA probes and their use for hygienic drinking water control. *Water Sci. Technol.,* 1997, vol. 35, 437-444
- **REX, J. H. ; M. A. PFALLER ; J. N. GALGIANI ; M. S. BARTLETT ; A. ESPINEL-INGROFF ; M. A. GHANNOUM ; M. LANCASTER ; F. C. ODDS ; M. G. RINALDI ; T. J. WALSH.** Development of interpretive breakpoints for antifungal susceptibility testing: conceptual framework and analysis of in vitro-in vivo correlation data for fluconazole, itraconazole, and candida infections. Subcommittee on Antifungal Susceptibility Testing of the National Committee for Clinical Laboratory Standards [see comments. *Clin Infect Dis.,* 1997, vol. 24, 235-47
- **ROLLER, C. ; M. WAGNER ; R. AMANN ; W. LUDWIG ; K. H. SCHLEIFER.** In situ probing of gram-positive bacteria with high DNA G + C content using 23S rRNA-targeted oligonucleotides. *Microbiology,* Mai 1995, vol. 141 (5), 1267
- *Microbiology,* vol. 140, 2849-58
- **ROSENTHAL, E. J. K.** Septikämie-Erreger 1983-1985. Ergebnisse einer multizentrischen Studie. *Dtsch Med Wochenschr.,* 1986, vol. 49, 1-8

- **SANDHU, G. S. ; B. C. KLINE ; L. STOCKMAN ; G. D. ROBERTS.** Molecular probes for diagnosis of fungal infections. *J Clin Microbiol,* Mai 1996, vol. 34 (5), 1350
- *J Clin Microbiol.,* vol. 33, 2913-9
- **SCHLEIFER, K. H. ; O. KANDLER.** Peptidoglycan types of bacterial cell walls and their taxonomic implications. *Bacteriol Rev.,* 1972, vol. 36, 407-77
- **SCHONHUBER, W. ; B. FUCHS ; S. JURETSCHKO ; R. AMANN.** Improved sensitivity of whole-cell hybridization by the combination of horseradish peroxidase-labeled oligonucleotides and tyramide signal amplification. *Appl Environ Microbiol.,* 1997, vol. 63, 3268-73
- **SOGIN, S. J. ; M. L. SOGIN ; C. R. WOESE.** Phylogenetic measurement in procaryotes by primary structural characterization. *J Mol Evol.,* 1971, vol. 1, 173-84
- Development and application of nucleic acid probes. **STAHL, D. A. ; R. AMANN.** Nucleic Acid Techniques in bacterial systematics. John Wiley & sons, 1991, 205-248
- **STAMENKOVIC, I. ; P. D. LEW.** Early recognition of potentially fatal necrotizing fasciitis. The use of frozen-section biopsy. *N Engl J Med.,* 1984, vol. 310, 1689-93
- **TREBESIUS, K. ; R. AMANN ; W. LUDWIG ; K. MÜHLEGGER ; K.-H. SCHLEIFER.** Identification of whole fixed bacterial cells with nonradioactive 23S rRNA-targeted polynucleotide probes. *Appl Environ Microbiol.,* 1994, vol. 60, 3228-3235
- **TREBESIUS, K. ; D. HARMSEN ; A. RAKIN ; J. SCHMELZ ; J. HEESEMANN.** Development of rRNA-targeted PCR and in situ hybridization with fluorescently labelled oligonucleotides for detection of Yersinia species. *J Clin Microbiol.,* 1998, vol. 36, 2557-64
- **TÜMMLER, B. ; C. KIEWITZ.** ystic fibrosis: an inherited suseptibility to bacterial respiratory infections. *Mol. Med. Today.,* 1999, vol. 5, 351-358
- **VAN DE PEER, Y. ; E. ROBBRECHT ; S. DE HOOG ; A. CAERS ; P. DE RIJK ; R. DE WACHTER.** Database on the structure of small subunit ribosomal RNA. *Nucleic Acids Res.,* 1999, vol. 27, 179-83
- **WALLNER, G. ; R. AMANN ; W. BEISKER.** Optimizing fluorescent in situ hybridization with rRNA-targeted oligonucleotide probes for flow cytometric identification of microorganisms. *Cytometry,* 1993, vol. 14, 136-43
- **WETMUR, J. G.** DNA probes: applications of the principles of nucleic acid hybridization. *Crit Rev Biochem Mol Biol.,* 1991, vol. 26, 227-59
- **ZACH, M. S.** Lung disease in cystic fibrosis--an updated concept. *Pediatr Pulmonol.,* 1990, vol. 8, 188-202
- **ZARDA, B. ; R. AMANN ; G. WALLNER ; K. H. SCHLEIFER.** Identification of single bacterial cells using digoxigenin-labelled, rRNA-targeted oligonucleotides. *J Gen Microbiol.,* 1991, vol. 137, 2823-30

SEQUENZPROTOKOLL

<110> Creatogen GmbH

<120> Verfahren der Ganzzellhybridisierung

<130> 21322P WO

<140>
<141>

<150> DE 199 55 303.3
<151> 1999-11-17

<160> 43

<170> PatentIn Ver. 2.1

<210> 1
<211> 18
<212> DNA
<213> Eubakterien

<400> 1

gctgcctccc gtaggagt          18

<210> 2
<211> 17
<212> DNA
<213> Prevotella + Bacteroides

<400> 2
ccaatgtggg ggacctt          17

<210> 3
<211> 18
<212> DNA
<213> Enterobacteriaceae

<400> 3
cccccwcttt ggtcttgc          18

<210> 4
<211> 18
<212> DNA
<213> Streptococcaceae

<400> 4
ccccttgtgt aaggcagg          18

<210> 5
<211> 18
<212> DNA
<213> Staphylococcaceae

<400> 5
tcctccatat ctctgcgc          18

<210> 6
<211> 18
<212> DNA
<213> Streptococcaceae

<400> 6
cactctcccc ttctgcac          18

<210> 7
<211> 20
<212> DNA
<213> Clostridium perfringens

<400> 7
ggttgaatga tgatgccatc          20

<210> 8
<211> 18
<212> DNA
<213> Peptostreptococcus anaerobius

<400> 8
ttatcgacgc caccttcg          18

<210> 9
<211> 19
<212> DNA
<213> Peptostreptococcus magnus

<400> 9
ccaatgcttt acggggttg          19

<210> 10
<211> 18
<212> DNA
<213> Peptostreptococcus micros

<400> 10
catgcgattc tgtggtct          18

<210> 11
<211> 19
<212> DNA
<213> Pseudomonas aeruginosa

<400> 11
gctgaatcca ggagcaagc          19

<210> 12
<211> 18
<212> DNA
<213> Pseudomonas aeruginosa

<400> 12
ggtaaccgtc ccccttgc          18

<210> 13
<211> 19
<212> DNA
<213> Staphylococcus aureus

<400> 13
gaagcaagct tctcgtccg          19

<210> 14
<211> 20
<212> DNA
<213> Streptococcus agalactiae

<400> 14
gtaaacacca aacmtcagcg          20

<210> 15
<211> 20
<212> DNA
<213> Streptococcus pyogenes

<400> 15
ctaacatgcg ttagtctctc          20

<210> 16
<211> 19

<212> DNA
<213> Streptococcus pyogenes

<400> 16
tccaaagcgt acattggtt          19

<210> 17
<211> 18
<212> DNA
<213> Enterococcus faecalis

<400> 17
ccctctgatg ggtaggtt          18

<210> 18
<211> 19
<212> DNA
<213> Candida albicans

<400> 18
gccaaggctt atactcgct          19

<210> 19
<211> 19
<212> DNA
<213> Candida glabrata

<400> 19
ccgccaagcc acaaggact          19

<210> 20
<211> 18
<212> DNA
<213> Candida krusei

<400> 20
gattctcggc cccatggg          18

<210> 21
<211> 19
<212> DNA
<213> Candida parapsilosis

<400> 21
cctggttcgc caaaaaggc          19

<210> 22
<211> 18
<212> DNA
<213> Enterokokken

<400> 22
ccccttctga tgggcagg          18

<210> 23
<211> 18
<212> DNA
<213> Klebsiella pneumoniae

<400> 23
cctacacacc agcgtgcc          18


<210> 24
<211> 19
<212> DNA
<213> Stenotrophomonas maltophilia


<400> 24
gtcgtccagt atccactgt         19


<210> 25
<211> 18
<212> DNA
<213> Streptococcus pneumoniae


<400> 25
gtgatgcaag tgcacctt          18


<210> 26
<211> 18
<212> DNA
<213> Genus Burkholderia


<400> 26
atggtcggaa cagagggt          18


<210> 27
<211> 18
<212> DNA
<213> Burkholderia cepacia


<400> 27
ctgtgcgccg gttctctt          18


<210> 28
<211> 18
<212> DNA
<213> Haemophilus influenzae


<400> 28
ccgcactttc atcttccg          18


<210> 29
<211> 18
<212> DNA
<213> Chlamydia


<400> 29
ctcaatccgc ctagacgt          18


<210> 30
<211> 18
<212> DNA
<213> Chlamydia


<400> 30
gctccccttg ctttcgcg          18

<210> 31
<211> 18
<212> DNA
<213> Gardnerella vaginalis

<400> 31
caccatgaag caacccgt        18


<210> 32
<211> 18
<212> DNA
<213> Ureaplasma urealyticum

<400> 32
gttccccaac tccctact        18


<210> 33
<211> 18
<212> DNA
<213> Chlamydia trachomatis

<400> 33
tcggatgccc aaatatcg        18


<210> 34
<211> 18
<212> DNA
<213> Chlamydia trachomatis

<400> 34
attagatgcc gactcggg        18


<210> 35
<211> 18
<212> DNA
<213> Pseudomonas aeruginosa

<400> 35
attgctaacc tcgctcga        18


<210> 36
<211> 18
<212> DNA
<213> Pseudomonas aeroginosa

<400> 36
tctcggcctt gaaacccc        18


<210> 37
<211> 20
<212> DNA
<213> Steptococcus pyogenes

<400> 37
ttccaaagcg tacattggtt        20


<210> 38
<211> 19

<212> DNA
<213> Hefen

<400> 38
ctctggcttc accctattc        19

<210> 39
<211> 18
<212> DNA
<213> Enterococcus faecium

<400> 39
cacacaatcg taacatcc        18

<210> 40
<211> 18
<212> DNA
<213> Escherichia coli + Shigella

<400> 40
accgtgaggc ttaacctt        18

<210> 41
<211> 18
<212> DNA
<213> Neisseria meningitidis

<400> 41
atatcagcgg ctgatgac        18

<210> 42
<211> 18
<212> DNA
<213> Listeria monocytogenes

<400> 42
ataagatgtg gcgcatgc        18

<210> 43
<211> 15
<212> DNA
<213> Pseudomonaceae

<400> 43
gctggcctag ccttc        15